(19) Europäisches Patentamt / European Patent Office / Office européen des brevets

(11) **EP 3 252 461 B1**

(12) **EUROPEAN PATENT SPECIFICATION**

(45) Date of publication and mention of the grant of the patent:
**26.05.2021 Bulletin 2021/21**

(51) Int Cl.:
*G01N 27/74* (2006.01)    *G01N 33/543* (2006.01)

(21) Application number: **16305639.3**

(22) Date of filing: **02.06.2016**

(54) **A MAGNETIC MEASUREMENT SYSTEM BASED ON AN ULTRASENSITIVE PLANAR HALL MAGNETORESISTIVE BIOSENSOR (PHR) AND A METHOD FOR MEASURING LOW SPECIFIC BIOPARTICLES CONCENTRATIONS AND QUANTIFYING BIO-PARTICLES INTERACTIONS**

MAGNETISCHES MESSSYSTEM AUF BASIS EINES HOCHEMPFINDLICHEN PLANAREN HALL MAGNETORESISTIVEN BIOSENSORS (PHR) UND VERFAHREN ZUR MESSUNG NIEDRIGER SPEZIFISCHER BIOPARTIKELKONZENTRATIONEN UND ZUR QUANTIFIZIERUNG VON BIOPARTIKELINTERAKTIONEN

SYSTÈME DE MESURE MAGNÉTIQUE ULTRASENSIBLE BASÉ SUR UN BIOCAPTEUR PLANAIRE À EFFET HALL (PHR) ET PROCÉDÉ POUR MESURER DE FAIBLES CONCENTRATIONS DE BIOPARTICULES SPÉCIFIQUES ET QUANTIFIER DES INTERACTIONS DE BIO-PARTICULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.12.2017 Bulletin 2017/49**

(73) Proprietors:
• **UNIVERSITE DE MONTPELLIER**
  **34090 Montpellier (FR)**
• **Centre National de la Recherche Scientifique**
  **75016 Paris (FR)**
• **Daegu Gyeongbuk Institute of Science and Technology**
  **Daegu 711-873 (KR)**
• **Ecole Nationale Supérieure de Chimie de Montpellier**
  **34296 Montpellier (FR)**

(72) Inventors:
• **TRAN, Quang Hung**
  **34270 Le Triadou (FR)**
• **TERKI, Ferial**
  **34090 Montpellier (FR)**
• **KAMARA, Souleymane**
  **34090 Montpellier (FR)**
• **FELIX, Gautier**
  **34090 Montpellier (FR)**
• **KIM, CheolGi**
  **Daegu 704-843 (KR)**
• **KIM, KunWoo**
  **Daegu 42996 (KR)**
• **KIM, SungJoon**
  **Daegu (KR)**
• **RAMULU, Torati Sri**
  **Daegu (KR)**

(74) Representative: **Marks & Clerk France**
**Immeuble "Visium"**
**22, avenue Aristide Briand**
**94117 Arcueil Cedex (FR)**

(56) References cited:
**US-A1- 2015 168 507**

• **BIN DENG ET AL.: "Aptamer binding assays for proteins: the thrombin example - A review", ANALYTICA CHIMICA ACTA, vol. 837, 21 July 2014 (2014-07-21), pages 1-15, XP028862975, ELSEVIER, AMSTERDAM, NL DOI: 10.1016/j.aca.2014.04.055**
• **B. SINHA ET AL: "Planar Hall magnetoresistive aptasensor for thrombin detection", BIOSENSORS AND BIOELECTRONICS, vol. 59, 27 March 2014 (2014-03-27), pages 140-144, XP055322555, NL ISSN: 0956-5663, DOI: 10.1016/j.bios.2014.03.021**

- **SUNJONG OH ET AL: "Hybrid AMR/PHR ring sensor", SOLID STATE COMMUNICATIONS, PERGAMON, GB, vol. 151, no. 18, 29 May 2011 (2011-05-29) , pages 1248-1251, XP028251303, ISSN: 0038-1098, DOI: 10.1016/J.SSC.2011.05.049 [retrieved on 2011-06-12]**
- **SUNJONG OH ET AL: "Analytes kinetics in lateral flow membrane analyzed by cTnI monitoring using magnetic method", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 160, no. 1, 19 August 2011 (2011-08-19), pages 747-752, XP028110965, ISSN: 0925-4005, DOI: 10.1016/J.SNB.2011.08.058 [retrieved on 2011-09-08]**

**Description**

TECHNICAL FIELD OF THE INVENTION

The present invention concerns a magnetic measurement system based on an ultrasensitive Planar Hall magnetoresistive biosensor(s) for measuring low concentrations down to femtomoles of specific bio-particles contained in a solution and/or quantifying the said bio-particles interactions.

The invention also relates to a method using the magnetic measurement system according to the invention for measuring low concentrations down to femtomoles of bio-particles contained in a solution and quantifying the interactions of the specific bio-particles, in particular for concentrations ranging from nano-moles to pico-moles wherein the Van der Waals repulsive forces have to be taken account.

BACKGROUND OF THE INVENTION

Aptamers are short strands of nucleotides (single strand DNA and RNA) that can bind with high affinity and specificity to targets ranging from cells to proteins and from proteins to small molecules. Usually, aptamers have been selected for various targets from a large random sequence pool and have been created by an *in vitro* process that includes repeated rounds of binding, partition and amplification and that is known as systematic evolution of ligands by exponential enrichment (SELEX).

As complements to antibodies, aptamers exhibit several unique features, including ease chemical modification, reversible folding, and excellent stability, which is beneficial to the development of affinity binding assays. Aptamers play a significant role in disease research and clinical diagnosis, due to the long term stability of the DNA probe.

Binding of thrombin and its aptamers in form of short strands of nucleotides is widely used as a proof-of-concept model in the development and demonstration of analytical methods because of their fast interaction, high affinity and specificity. Indeed, thrombin is a serine protease known as a key enzyme responsible for the conversion of fibrinogen to fibrin in the blood coagulation and wound healing processes. Under normal conditions, thrombin is present in its inactive form prothrombin in blood. After injury of vascular, thrombin is activated immediately to cleave fibrinogen and form clots preventing bleeding. Thrombin concentration plays a crucial role in physiological and pathological conditions, and is an essential marker for the diagnosis of various diseases, i.e. Alzheimer, cardiovascular disease, and cancers. Therefore, the development of devices and methods for the monitoring and detection of low bio-molecular concentrations, in particular low thrombin concentrations, with high sensitivity and selectivity is of enormous importance in medical applications.

Many various types of DNA-aptamer based techniques for thrombin detection have been used and are reviewed in the article from Bin Deng et al., entitled "Aptamer binding assays for proteins: the thrombin example - A review", published in Analytica Chimica Acta 837 (2014) 1-15. Among the DNA-aptamer based techniques that have been used in thrombin detection, fluorescence, surface plasma resonance, quartz crystal microbalance and electrochemistry can be cited.

In order to improve the sensitivity of the detection of specific bio-particles and to detect lower concentrations of said bio-particles, the use of magnetic labels coupled with magneto-resistive (MR) sensors has been proposed. Moreover, MR sensors are low-power consumptions sensors, they are highly sensitive, easily scalable, inexpensive and portable. Several types of MR sensors, such as semiconductor Hall sensor, anisotropic magnetoresistive (AMR), giant magnetoresistive (GMR), tunneling magnetoresistive (TMR) and giant magneto-impedance (GMI) sensors for bio-sensing application, especially detection of lower thrombin molecular concentrations have been described in several papers.

As an alternative to the MR sensors described here above and as a promising solution to detect lower concentrations of specific bio-particles, the use of Planar Hall Magnetoresistive (PHR) sensors has been proposed and described in several articles :

- the article of Sunjong Oh et al., entitled " Hybrid AMR/PHR ring sensor" published in Solid State Communications, Pergamon, GB, vol. 151, n° 18, 29 May 2011, pages 1248-151,
- the article Sunjong Oh et al., entitled "Analytes kinetics in lateral flow membrane analyzed by cTNI monitoring using magnetic method", published in Sensors ad Actuators B: Chemical International : International journal devoted to research and development of physical and chemical transducers, Elsevier BV, NL, Vol. 160 n° 1, 19 August 2011, pages 747-752,
- the patent document US 2015/168507 A1,*
- the article from P. Sinha et al. entitled "Planar Hall magnetoresistive aptasensor for thrombin detection", published in Biosensors and Bioelectronics 59 (2014) 140-144.

The article from Sinha et al. describes the development of a sensitive aptamer-based PHR sensor for human α-thrombin detection. The sensing mechanism is based on the sandwich-type aptamer assay. The sensor surface is modified with thiol-labeled primary aptamer, and then thrombin is applied to the aptamer immobilized surface. After that, biotin-labeled secondary aptamer is used to bind with thrombin. Finally, streptavidin-coated magnetic nanoparticles are used to measure the MR signal. The MR signal is measured by the changes in the thrombin concentration which results in the changes in the amount of biotin-labeled secondary aptamer for the binding of streptavidin-coated magnetic nanoparticles in the assay. A minimum concentration detection of 86 pM has been achieved with this aptamer-based PHR sensor.

A first technical problem is to provide a magnetic measurement system based on an ultrasensitive Planar Hall magnetoresistive biosensor and a corresponding method for measuring low concentrations of specific bio-particles that improves the minimum concentration detection level or the minimum concentration detection threshold of the said specific bio-particles contained in a given solution in view of the existing magnetic measurement system and measurement methods based on ultrasensitive Planar Hall magnetoresistive biosensor(s).

A second technical problem, connected to the first technical problem, is to provide a method for quantifying interactions of specific bio-particles contained in a given solution, in particular in the solutions having concentrations ranging from nano-moles to pico-moles and wherein the Van der Waals repulsive forces have to be taken account.

SUMMARY OF THE INVENTION

The invention aims at solving the first technical problem and the second technical problem.

To this end, the invention relates to a magnetic measurement system for measuring a concentration of specific bio-particles through a sensitive detection of magnetic labeled particle(s), the magnetic measurement system comprising:

- an aptamer-based magnetic hybrid AMR/PHR sensor having :

  - an active surface on which at least one magnetic particle is ready to be or is closely bound through a sandwich-type aptamer structure that includes a captured unitary bio-particle, the active surface including a magnetic track with a closed loop shape,
  - a first current terminal and a second terminal, forming a pair of current terminals facing each other and contacting with the closed loop magnetic track,
  - a first voltage terminal and a second voltage terminal forming a pair of voltage terminals facing each other and contacting with the closed loop magnetic track,

  - an electrical bias current source configured to inject a bias current in the magnetic track, the electrical source being connected between the first and second current terminals, and
  - a voltage measurement device connected between the first and second voltage terminals measuring a differential voltage $V_{MR}$ between the pair of voltage terminals,
  - a magnetic element for applying an actual DC constant magnetic field on the magnetic track at a predetermined amplitude;
  - a signal processing device configured to deduce at least one physical quantity based on the differential voltage and representative of the bio-particles concentration;

the magnetic measurement system being characterized in that the actual amplitude of the applied DC constant magnetic field is closely located in a vicinity of an optimum amplitude of the permanent DC magnetic field comprised in a set of two optimum values H1, H2 of the permanent DC magnetic field, each optimum value H1, H2 maximizing locally the

$$H \cdot \frac{\partial V_{MR}}{\partial H},$$

absolute value of a first quantity

where H is the amplitude of the applied magnetic field and $\dfrac{\partial V_{MR}}{\partial H}$ is the derivate of the differential voltage with respect to the magnetic field at the applied field H.

According to further aspects of the invention which are advantageous but not compulsory, the magnetic measurement system might incorporate one or several of the following features, taken in any technically admissible combination:

   .- the diameter of the vicinity surrounding the optimum amplitude H1 and H2, of the permanent DC magnetic field is lower than a first predetermined threshold so that a first ratio of the absolute value of the first quantity $H \cdot \dfrac{\partial V_{MR}}{\partial H}$ at the actual amplitude of the applied DC constant magnetic field over the maximum absolute value of the quantity $H \cdot \dfrac{\partial V_{MR}}{\partial H}$ at the corresponding optimum amplitude H1 or H2 is lower than minus 3 dB;

.- the local optimum amplitude H2 of the permanent DC magnetic field closest to the actual amplitude of the applied DC constant magnetic field is selected among the two optimum amplitudes H1, H2 of the set as the local optimum amplitude that maximizes a second quantity $H \cdot \dfrac{\partial V_{MR}}{\partial H} / BN(H)$ defined as the ratio of the first quantity $H \cdot \dfrac{\partial V_{MR}}{\partial H}$ to the Barkhausen noise level BN(H) at the applied field, the Barkhausen noise being proportional to $\dfrac{dM}{\partial H}(H))$, where M is the magnetization of the magnetic sensor;

.- the diameter of the vicinity surrounding the optimum amplitude H1 or H2 of the permanent DC magnetic field is lower than a second predetermined threshold so that a second ratio of the absolute value of a second quantity $H \cdot \dfrac{\partial V_{MR}}{\partial H} / BN(H)$ at the actual amplitude of the applied DC constant magnetic field over the maximum absolute value of the second quantity $H \cdot \dfrac{\partial V_{MR}}{\partial H} / BN(H)$ at the corresponding optimum amplitude H1 or H2 is lower than minus 3 dB, the second quantity $H \cdot \dfrac{\partial V_{MR}}{\partial H} / BN(H)$ being defined as the ratio of the first quantity $H \cdot \dfrac{\partial V_{MR}}{\partial H}$ to the Barkhausen noise level BN(H) at the applied field, the Barkhausen noise being proportional to $\dfrac{dM}{\partial H}(H))$, where M is the magnetization of the magnetic sensor;

.- the magnetic measurement system as defined here above comprises further an AC field magnetic source for applying an actual AC alternative magnetic field on the magnetic track at a predetermined amplitude and at a predetermined frequency, the applied actual AC alternative magnetic field being collinear to the actual constant DC magnetic field applied by the magnetic element;

.- the AC field magnetic source is either an external magnetic field source including at least one coil and a coil current source for supplying an AC current source, or is an electrical AC bias current source, configured to inject an alternative AC bias current in the magnetic track, the magnetic track generating accordingly by self induction the applied AC field;

.- the magnetic element is a set comprising at least one magnetic coil or a permanent magnet;

.- the magnetic track is chosen in the group consisting of a cross track, a ring track and a multi-ring track;

.- the magnetic track is a ring-track, the number of rings being comprised between 1 and 17;

.- the magnetic track is made in a structure chosen in the group consisting of a tri-layered structure including a ferromagnetic film, a metal and an anti-ferromagnetic film, in particular Ta/NiFe/Cu/IrMn/Ta, a bilayer structure, in particular Ta/NiFe/IrMn/Ta, and a spin-valve, in particular Ta/NiFe/Cu/NiFe/IrMn/Ta;

.- the signal processing device comprises a standalone processor when no AC magnetic field is applied onto the magnetic track (24), and comprises a processor and a lock-in unit when an AC magnetic field is applied onto the magnetic track; and the signal processing device is configured to extract from the differential voltage $V_{MR}$ the first or second harmonic of the differential voltage when a an AC magnetic field is applied onto the magnetic track; and the signal processing is configured to determine said at least one physical quantity based on said first or second harmonic, when the electrical bias current source injects an alternative AC bias current in the magnetic track or when an external AC magnetic fied is applied collinearly with the external constant DC magnetic field.

The invention also relates to a magnetic measurement method for measuring low concentrations of bio-particles contained in a given solution and/or quantifying the interactions of the said specific bio-particles contained in a given solution, in particular in solutions having concentrations ranging from nano-moles to pico-moles, the magnetic measuring method comprising the steps of:

- providing a magnetic measurement system configured for measuring a concentration of bio-particles through a sensitive detection of magnetic particle labels, the magnetic measurement system comprising:
- an aptamer-based magnetic hybrid AMR/PHR sensor including :

  • a prepared active surface on which at least one magnetic particle is ready to be or is closely bound through a sandwich-type aptamer structure that includes a captured unitary bio-particle, the active surface including a magnetic track with a closed loop shape,
  • a first current terminal and a second terminal, forming a pair of current terminals facing each other and contacting with the closed loop magnetic track,
  • a first voltage terminal and a second voltage terminal forming a pair of voltage terminals facing each other and contacting with the closed loop magnetic track,

  - injecting a bias current in the magnetic track by using the electrical source being an electrical bias current source connected between the first and second current terminals, and
  - applying an actual DC constant magnetic field on the magnetic track at a predetermined amplitude by using a magnetic element;
  - measuring a differential voltage $V_{MR}$ between the pair of voltage terminals by using a voltage measurement device connected between the first and second voltage terminals,

- determining at least one physical quantity based on the differential voltage and representative of the bio-particles concentration by using a signal treatment device ; the said magnetic measurement method being characterized in that the actual amplitude of the applied DC constant magnetic field is closely located in a vicinity of an optimum amplitude of the permanent DC magnetic field comprised in a set of two optimum values H1, H2 of the permanent DC magnetic field, each optimum value H1, H2 maximizing locally the absolute value of a first quantity $H \cdot \dfrac{\partial V_{MR}}{\partial H}$,

where H is the amplitude of the applied magnetic field and $\dfrac{\partial V_{MR}}{\partial H}$ is the derivate of the differential voltage with respect to the magnetic field at the applied field H.

According to further aspects of the invention which are advantageous but not compulsory, the magnetic measurement method might incorporate one or several of the following features, taken in any technically admissible combination:

- the magnetic measuring method as defined here above comprises a step of applying on the prepared active surface a drop of a magnetic solution containing streptavidin-coated magnetic nanoparticles that is executed , either before the step of providing the magnetic measurement system when measuring the concentration of the bio-particles, or after the start of the steps of injecting a bias current in the magnetic track and applying a DC constant magnetic field onto the magnetic track and after the start of the step of measuring a differential voltage $V_{MR}$ when quantifying the interactions of the specific bio-molecules;
- the local optimum amplitude H2 of the permanent DC magnetic field closest to the actual amplitude of the applied DC constant magnetic field is selected among the two optimum amplitudes H1, H2 of the set as the local optimum amplitude that maximizes a second quantity $H \cdot \dfrac{\partial V_{MR}}{\partial H} / BN(H)$ defined as the ratio of the first quantity

$H \cdot \dfrac{\partial V_{MR}}{\partial H}$ to the Barkhausen noise level BN(H) at the applied field, the Barkhausen noise being proportional to

$\dfrac{dM}{\partial H}(H))$, where M is the magnetization of the magnetic sensor;

- the magnetic measuring method as defined here above comprises a step of preparing the active surface by modifying the active surface with thiol-labeled primary aptamer, then applying a drop of a solution of specific bio-particles at a concentration to be determined to the primary immobilized surface, the volume of the drop being a priori known, then using biotin-labeled secondary aptamers to bind with the bio-particles;
- the magnetic measuring method as defined here above for quantifying the interactions of specific bio-particles contained in a given solution comprises a supplemental signal processing step including the steps of:
- determining the kinetics of the binding of the magnetic particles to the specific bio-particles captured and immobilized on the active surface is determined from the temporal evolution of the measured magnetic signal; then
- determining a saturation time from the binding kinetics of the magnetic particles;
- from the saturation time and a model function, computing an average distance separating the bio-particles in their solution and quantifying the interactions of specific bio-particles that break down into a long-range Coulomb interaction and a short-range Van de Waals interaction.

BRIEF DESCRIPTION OF THE DRAWINGS

The invention will be better understood on the basis of the following description which is given in correspondence with the annexed figures and as an illustrative example, without restricting the object of the invention. In the annexed figures:

- Figure 1 is view of a measurement device according to a first embodiment of the invention that comprises a magnetic AMR/PHR biosensor,
- Figure 2 is a view of an exemplary prepared active surface covered by immobilized aptamer sandwich structure embedding a specific bio-particle, here thrombin, and a magnetic label particle to be bound to the immobilized aptamer sandwich structure;
- Figure 3 is a planar detailed view of the AMR/PHR multi-ring magnetic AMR/PHR biosensor of figure 1,
- Figure 4 are plots illustrating the field-sensitivity of an AMR/PHR sensor, the stray field of the magnetic particles, and the planar Hall voltage change with respect to magnetic particles as a function of the applied magnetic field;
- Figure 5 are plots illustrating the Barkhausen noise as a function of the applied magnetic field,
- Figure 6 is a view of a measurement device according to a first variant of a second embodiment of the invention that comprises a magnetic AMR/PHR aptasensor,
- Figure 7 is a view of a measurement device according to a second variant of the second embodiment of the invention that comprises a magnetic AMR/PHR biosensor,
- Figure 8 is a flow chart of a magnetic measurement method according to the invention for measuring low concentrations of specific bio-particles and/or quantifying the interactions of the said specific bio-particles, carried out by the magnetic measurement system of the Figures 1, 6 and 7;
- Figure 9 is a detailed flow chart of a magnetic measurement method according to the invention for quantifying the interactions of specific bio-particles of a given solution;
- Figure 10 is a view of a model of the interactions existing between thrombin molecules contained in a solution at a given concentration;
- Figure 11 is a plot of the voltage change of the AMR/PHR biosensor a function of thrombin concentration;
- Figure 12 is a plot of real time voltage profiles for varying thrombin concentrations:
- Figure 13 is a plot of the threshold time as a function of thrombin concentration, and
- Figure 14 is a plot of the normalized voltage profile of the lowest and the highest concentration of thrombin.

DETAILED DESCRIPTION OF SOME EMBODIMENTS

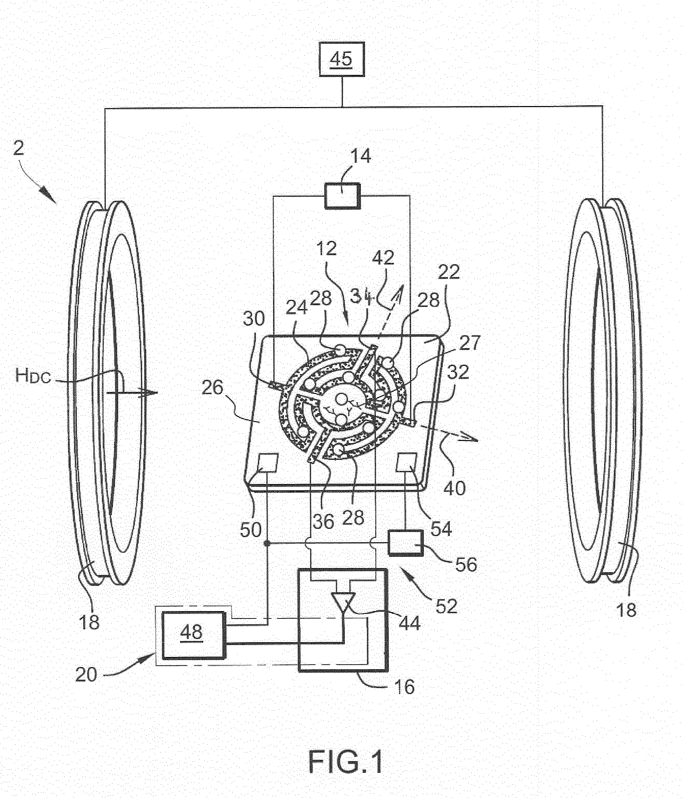

According to Figure 1 and a first embodiment of the invention, a magnetic measurement system 2, also called a micromagnetometry system, is configured for measuring low concentration of specific bio-particles through an ultrasensitive detection of magnetic particle labels.

The term "bio-particles" shall be broadly interpreted as a particles intervening in a biological process and having a small size ranging from the cell down to a single bio-molecule. The set of bio-particles includes the cells, the bacteria, the viruses, the bio-inspired architectures or assemblies, the bio-molecules from cell size to nano-size biological assemblies, the bio-labeled nanoparticles, the aptamer molecules, the genes/antigens, the antibodies, the vectorized nanoparticles, and the target bio-molecules. The set of bio-particles includes core/shell hybrid particles from cell size to nano-size, wherein the core or the shell or both are bio-labelled, wherein the core can be magnetic or bi-stable particle or silica gel or silica aerogel, the core, and wherein the shell can be a bio-inspired architecture and bio-hybrid particles.

In particular, the magnetic measurement system 2 is configured to detect the presence of at least one single magnetic label.

As representative examples, the magnetic particle labels are comprised in a set including metallic materials, metallic oxides, rare earth elements, organometallic complexes, coordination complexes (magnetic molecules, magnet chains).

The magnetic measurement system 2 comprises an aptamer-based magnetic hybrid AMR/PHR sensor 12, an electrical current source 14, a voltage measurement device 16, a magnetic element 18 and a signal treatment device 20.

The magnetic AMR/PHR sensor 12 is named sensor 12 in the remainder of the description. The sensor 12 is a sensor adapted to detect AMR and PHR, PHR being an acronym for Planar Hall Resistance.

The Planar Hall Resistance is based on the planar Hall effect of ferromagnetic materials. It measures the change in anisotropic magnetoresistance caused by an external magnetic field in the Hall geometry. The sensor responds to magnetic field components in the sensor plane as opposed to the ordinary Hall sensor, which measures field components perpendicular to the sensor plane. Hence, the used name "planar Hall". Generally speaking, for ferromagnetic materials, the resistance is larger when the current flows along the direction of magnetization than when it flows perpendicularly to the magnetization vector. This creates an asymmetric electric field perpendicular to the current, which depends on the magnetization state of the sensor.

AMR is an acronym for Anisotropic Magneto-Resistance. Magneto-resistance is the property of a material to change the value of the electrical resistance of the material when an external magnetic field is applied to the material. More specifically, anisotropic magnetoresistance is a property of a material in which a dependence of electrical resistance on the angle between the direction of electric current and direction of magnetization is observed. The effect arises from the simultaneous action of magnetization and spin-orbit interaction and its detailed mechanism depends on the material. It can be for example due to a larger probability of s-d scattering of electrons in the direction of magnetization (which is controlled by the applied magnetic field). The net effect (in most materials) is that the electrical resistance has maximum value when the direction of current is parallel to the applied magnetic field.

The sensor 12 comprises an active surface 22 that includes a magnetic track 24 with a closed loop shape deposited on a substrate 26. The active surface 22 has been previously prepared by covering the said surface 22 by sandwich-type aptamer structures 27 that include individually a specific captured bio-molecule. To achieve a prepared active surface, the active surface has been firstly modified with thiol-labeled primary aptamer, and then specific bioparticles contained in a solution at a concentration to be determined are applied to the aptamer immobilized surface. After that, biotin-labeled secondary aptamers are used to bind with the specific bio-molecules.

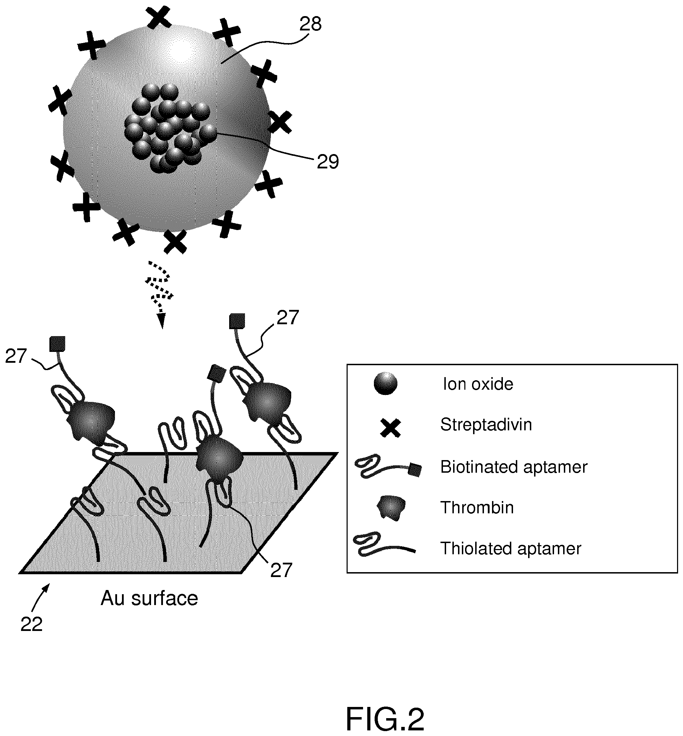

Thus, the active surface 22 is ready to bind streptavidin-coated magnetic particles forming magnetic labels 28 to the sandwich-type aptamer structures 27 as shown in Figure 2 wherein the specific bioparticle is thrombin as an example.

According to Figure 2, a magnetic label or particle 28, drawn to scale, comprises a dozen or more iron-oxide cores 29 embedded in a dextran polymer functionalize with Streptadivin.

When measuring only the concentration of the specific bio-particles is sought, the magnetic labels 28 may be bound to the sandwich-type aptmer structures 27 before the measurement is carried out.

When quantifying the interactions of the specific bio-particles is sought, the magnetic labels 28 shall be applied to the sandwich-type aptamer structures 27 covering the active surface when the magnetic MR signal measurement has started since determining the saturation time from the binding kinetics of the magnetic particles is needed.

The magnetic labels or magnetic particles 28 to be detected are motionless and placed close to the active surface 22 of the sensor 12 when they are bound to the sandwich-type aptamer structures 27.

Accordingly, when the magnetic measurement system 2 is used for the detection of specific bio-particles, for example thrombin, the magnetic particles 28 are bound to the specific bio-particles to be detected by means of secondary specific aptamers, the specific bio-particles being themselves bound to the active surface 22 and especially the magnetic track 24 by means of primary aptamers. Thus, the number of magnetic particles 28 on the magnetic track 28 equals the number of bio-particles to be detected, so the detection of the number of magnetic particles 28 on the magnetic track 24 allows determining the number of the specific bio-particles bound to the active surface 22.

According to the Figure 1 the sensor 12 has a first current terminal 30 and a second current terminal 32 forming a pair of terminals which face each other contacting with the closed loop magnetic track 24.

The sensor 12 also has a first voltage terminal 34 and a second voltage terminal 36 forming a pair of terminals which face each other contacting with the closed loop magnetic track 24.

The sensor 12 also has a first axis 40 or easy axis passing through the first and second current terminals 30, 32 being parallel to the exchange bias field direction of the material and perpendicular to a second axis 42 or hard axis passing through the first and second voltage terminals 34, 36.

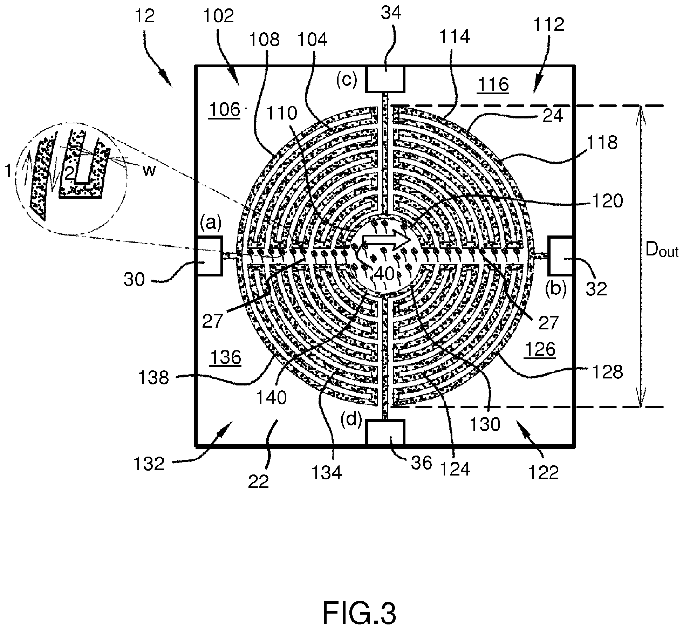

According to the Figure 3, the sensor 12 is based on a multi-ring architecture and is manufactured using a lithography technique in a clean-room of class 1000 with a lift-off process.

More specifically, the magnetic track 24 is a tri-layered material, including a ferromagnetic film, a metal and an anti-ferromagnetic film, for example Ta(5)/NiFe(10)/Cu(0.1)/IrMn(10)/Ta(5) (nm). In this structure, the soft magnetic layer NiFe is the sensing material that is weakly coupled to an anti-ferromagnetic layer (IrMn) by a long range exchange bias field through an atomic Cu sub layer.

The tri-layer structure Ta(5)/NiFe(10)/Cu(0.1)/IrMn(10)/Ta(5) nm is deposited by a 6 gun-magnetron sputtering system with a based vacuum of about $10^{-8}$ Torr.

To prevent the contamination and current leak, the magnetic sensor is passivated by a $Si_2O_3$ or $Si_3N_4/Si_2O_3$ bi-layer with a thickness of 200 nanometers.

The outer diameter of the sensor 12 is equal here to 300 $\mu$m and the width w of the magnetic track 24 is equal to 10 $\mu$m.

The magnetic track 24 of the sensor 12 has a first arm 102 made of a first set of a predetermined ring number m of circular meander paths 104 delimited within a first quarter surface 106 of the sensor 12, the outermost meander path 108 being connected to the first current terminal 30 and the innermost meander path 110 being connected to the first voltage terminal 34.

The ring number m is an integer comprised between 1 and 17.

The magnetic track 24 of the sensor 12 further comprises a second arm 112 made of a second set of the same predetermined number m of circular meander paths 114 delimited within a second quarter surface 116 of the sensor 12, the outermost meander path 118 being connected to the second current terminal 32 and the innermost meander path 120 being connected to the first voltage terminal 34.

The magnetic track 24 of the sensor 12 also comprises a third arm 122 made of a third set of the same ring number m of circular meander paths 124 delimited within a third quarter surface 126 of the sensor 12, the outermost meander path 128 being connected to the second current terminal 32 and the innermost meander path 130 being connected to the second voltage terminal 36.

The magnetic track 24 of the sensor 12 further comprises a fourth arm made 132 of a fourth set of the same ring number m of circular meander paths 134 delimited within a fourth quarter surface 136 of the magnetic sensor 12, the outermost meander path 138 being connected to the first current terminal 30 and the innermost meander path 140 being connected to the second voltage terminal 36.

This multi-ring architecture enhances the sensitivity of the sensor 12 in a compact region.

As the length of the arms increases with the ring number, filling the sensing meander paths enhances the active sensing area.

The current direction alternately changes for successive ring paths, i.e. there is a current angle range $\theta = \pi/2$ to 0 for path 1 shown in the inset of Figure 2, and $\theta = \pi$ to $3\pi/2$ for path 2 in the inset of Figure 3. The sign of the calculated value for path 1 and 2 is the same, which means that AMR effect for both currents is additive. Thus, the maximum voltage variation in the profiles and accordingly the field sensitivity of the arm resistance increases the ring number.

The voltage profile for the full magnetic ring is a linear response across zero field, where the AMR/PHR voltage of the ring are additive for all junction components

The sensitivity of first magnetic sensor is enhanced by using a tri-layer structure which has a small exchange coupling field and high active current.

This multi-ring architecture enhances the field sensitivity and the active area of the sensor 12.

Thus, the sensor 12 is a highly sensitive magneto-resistive (MR) sensor based on Planar Hall Effect (PHE) that is capable to detect the presence of magnetic particles 28 closely bound to the active surface 22 through the sandwich-type aptamer structures 27, to quantify therefore the number of immobilized magnetic particles 28, and to determine the concentration of bio-particles of the solution.

Preferably, the ring number m of circular meander paths is comprised between 9 and 17. Here, each arm has eleven meander paths or quarters of circular rings, this number maximizing the sensitivity of the sensor 12.

This multilayer stack exhibits a very high sensitivity of about S = 6 $V.T^{-1}$ and a low white noise of about 1 $nV \cdot Hz^{-1/2}$ at 100 Hz.

Here, the first electrical current or voltage source 14, connected between the first and second current terminals, is configured to inject a DC bias current for the sensor 12. The electrical DC bias current source is for example a Keithley 2400 current source and the amplitude of the DC bias current is set to 1 mA.

The voltage measurement device 16, connected between the first voltage terminal 34 and the second voltage terminal 36, is configured to measure the differential voltage $V_{MR}$ between the pair of voltage terminals 34, 36.

The voltage measurement device 16 comprises a low noise amplifier 44 with a gain of 20 dB for amplifying the detected differential voltage.

The magnetic element 18 is configured to apply a constant magnetic field $H_{DC}$ on the magnetic track 24. The magnetic element 18 is comprised in the set consisting of the single coils, the pair of Helmholtz coils, a plurality of coils, and the permanent magnets. Here the magnetic element 18 is a pair of Helmholtz coils supplied by a DC coil electrical source 45.

Generally, the actual amplitude H of the applied DC constant magnetic field by is chosen to be closely located in a vicinity of an optimum amplitude of the permanent DC magnetic field comprised in a set of two optimum values H1, H2 of the permanent DC magnetic field, each optimum value H1, H2 maximizing locally the absolute value of a first

quantity $H \cdot \dfrac{\partial V_{MR}}{\partial H}$, where H is the amplitude of the applied magnetic field and $\dfrac{\partial V_{MR}}{\partial H}$ is the derivate of the differential voltage with respect to the magnetic field at the applied field H.

The presence of the magnetic particles 28 on the magnetic track 24 results in a voltage change which depends on the number of magnetic particles 28 bound closely to the magnetic track 24. Especially, the magnetic particles 28 become magnetized when the external DC magnetic field is applied by the magnetic element 18. Their presence in close proximity of the sensor surface 22 alters the local magnetic field and consequently changes the output voltage of the magnetic sensor 12.

Indeed, the stray field $H_{stray}$ of the magnetic particles bound closely to the sensor 12 is proportional to the applied field according to the following equation:

$$H_{stray} = k \frac{n\chi V_{particle}}{4\pi z^3} H$$

where:

k designates a constant,
$\chi$ designates the magnetic susceptibility of the magnetic particles,
$V_{particle}$ designates the volume of a particle,
n the number of particles,
z the distance from any particle to the sensor 12,
H designates the applied magnetic field.

Thus, the differential voltage $V_{MR}$ measured by the voltage measurement device 14 changes with the number of the particles by an amount $\Delta V$ expressed by the equation:

$$\Delta V = S(H)\left(1 - k \frac{n\chi V_{particle}}{4\pi z^3}\right)H$$

where
S(H) designates the sensitivity of the sensor 12 at the applied DC field H.

Accordingly the change of the differential voltage can be also expressed by $\Delta V = \alpha \cdot H \cdot \dfrac{\partial V_{MR}}{\partial H}$. In this expression, $\alpha$ is a constant equal to $\left(1 - k \dfrac{n\chi V_{particle}}{4\pi z^3}\right)$, H is the constant DC magnetic field applied, $V_{MR}$ is the measured differential magnetoresistive voltage and $\dfrac{\partial V_{MR}}{\partial H}$ is the derivate of the magnetoresistive voltage with respect to the magnetic field at the actual applied field H, this derivate corresponding to the field-sensitivity i.e. the sensor sensitivity S(H).

Thus, in order to optimize the sensitivity of the measurement system 2 for magnetic particles detection, the amplitude of the actual amplitude H of the applied DC constant magnetic field by is chosen to be closely located in a vicinity of an optimum amplitude of the permanent DC magnetic field comprised in a set of two optimum values H1, H2 of the permanent DC magnetic field, each optimum value H1, H2 maximizing locally the absolute value of a first quantity $H \cdot \dfrac{\partial V_{MR}}{\partial H}$.

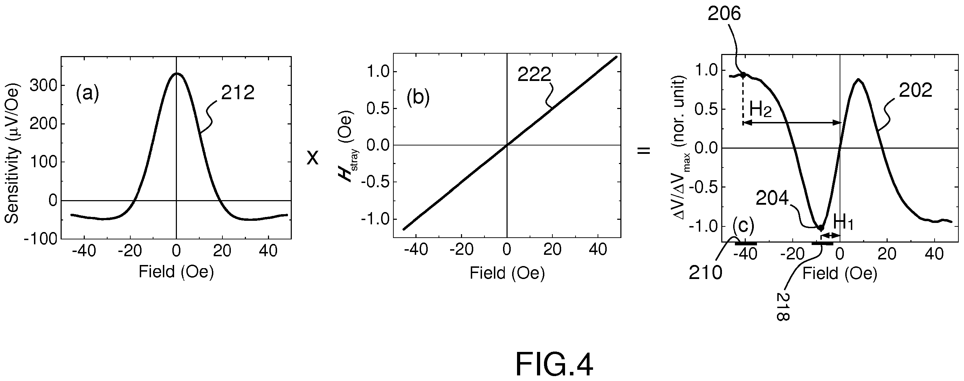

According to the Figure 4 and the view 4-(c), a first curve 202 of the evolution of the normalized first quantity $H \cdot \dfrac{\partial V_{MR}}{\partial H}$ (H), denoted $\Delta V / \Delta V_{max}$ in the Figure 4-(c), versus the actual applied field H, exhibits two maximal points 204, 206, here at amplitudes $H_1$=10 Oe and $H_2$=40 Oe in one magnetic direction for an exemplary case of multi-ring sensor 12.

According to the first curve 202, the sensor 12 has the highest sensitivity in detection of the magnetic particles 28 at these two maximal fields $H_1$ and $H_2$. The magnitudes of these two fields are dependent on the exchange bias $H_{ex}$ and coercivity fields $H_k$ of the sensor stack. Here, for this particular tested sensor, at these two fields $H_1$ and $H_2$, the voltage changes $\Delta V$ are equal. Accordingly, the amplitude of the constant magnetic field applied to the magnetic coil 18 is preferably selected around these values $H_1$ and $H_2$ in respective vicinities 218, 210.

Generally, the diameter of the vicinity 218, 210 surrounding the optimum amplitudes H1 and H2, of the permanent DC magnetic field is lower than a first predetermined threshold so that a first ratio of the absolute value of the first quantity

$$H \cdot \frac{\partial V_{MR}}{\partial H}$$

at the actual amplitude of the applied DC constant magnetic field over the maximum absolute value of the

quantity $H \cdot \frac{\partial V_{MR}}{\partial H}$ at the corresponding optimum amplitude H1 or H2 is lower than minus 3 dB.

As an example, the diameter of the vicinities 218 and 210 is chosen to be respectively equal to 10 and 20 Oe, the amplitude of the applied constant magnetic field being comprised between 5 Oe and 15 Oe, or between 30 Oe and 50 Oe.

According to the Figure 4, the first curve 202 can be constructed by multiplying a second curve 212, as shown in the view 4-(a), of the evolution S(H) of the sensitivity S of the sensor 12 versus the applied constant field H by a third curve 222, as shown in the view 4-(b), of the evolution the stray field of particles $H_{stray}$ versus the applied constant field H.

The second curve 212 is deduced from experimental measurement of the sensor carried out by using the exemplary test sensor as described in the "Test and performance results" section.

Generally the local optimum amplitude H2 of the permanent DC magnetic field closest to the actual amplitude of the applied DC constant magnetic field is selected among the two optimum amplitudes H1, H2 of the set as the local optimum amplitude that maximizes a second quantity $H \cdot \frac{\partial V_{MR}}{\partial H} / BN(H)$ defined as the ratio of the first quantity

$$H \cdot \frac{\partial V_{MR}}{\partial H}$$

to the Barkhausen noise level BN(H) at the applied field, the Barkhausen noise being proportional to

$$\frac{dM}{\partial H}(H)),$$

where M is the magnetization of the magnetic sensor.

The diameter of the vicinity surrounding the optimum amplitude H1 or H2 of the permanent DC magnetic field is lower than a second predetermined threshold so that a second ratio of the absolute value of a second quantity

$$H \cdot \frac{\partial V_{MR}}{\partial H} / BN(H)$$

at the actual amplitude of the applied DC constant magnetic field over the maximum absolute value of the second quantity $H \cdot \frac{\partial V_{MR}}{\partial H} / BN(H)$ at the corresponding optimum amplitude H1 or H2 is lower than minus 3 dB, the second quantity $H \cdot \frac{\partial V_{MR}}{\partial H} / BN(H)$ being defined as the ratio of the first quantity $H \cdot \frac{\partial V_{MR}}{\partial H}$ to the Barkhausen noise level BN(H) at the applied field, the Barkhausen noise being proportional to $\frac{dM}{\partial H}(H)),$ where M is the magnetization of the magnetic sensor.

Indeed, the noises of a biosensor are required to be minimized to increase the signal-to-noise (Su/N) ratio of the detection of magnetic particles 28 by the sensor 12, wherein the useful detection signal Su is the voltage change $\Delta V$ and N is the noise.

There are three main internal noise sources of the investigated sensor 12: 1/f noise, thermal noise and Barkhausen noise.

1/f noise can be minimized by using an alternative AC exciting field and by choosing an exciting frequency higher than the frequency knee.

Thermal noise of the sensor 12 having a diagonal configuration is smaller than other off-diagonal configuration sensors such as GMR, MTJ. The transverse resistivity is sensitive only to the anisotropic resistivity, and not to the isotropic resistivity term. Experimentally, noises of a PHE sensor is about four orders of magnitude smaller than the noise of a similar longitudinal anisotropic magnetoresistance (AMR) sensor only. Anisotropic resistance at H = 0 mT, $H_1$ = 1 mT and $H_2$ = 4 mT are 10 mΩ, 98.9 mΩ and 99.4 mΩ. The thermal noises of the sensor at these fields are calculated from $\Delta V_T = \sqrt{4 k_B T R \Delta f}$ to be 17 pV, 55.4 pV and 55.6 pV, respectively.

The Barkhausen noise at field H is proportional to $\dfrac{dM}{\partial H}$, i.e. the derivative of the magnetization M with respect to the magnetic field H applied for magnetization on the magnetic track 24.

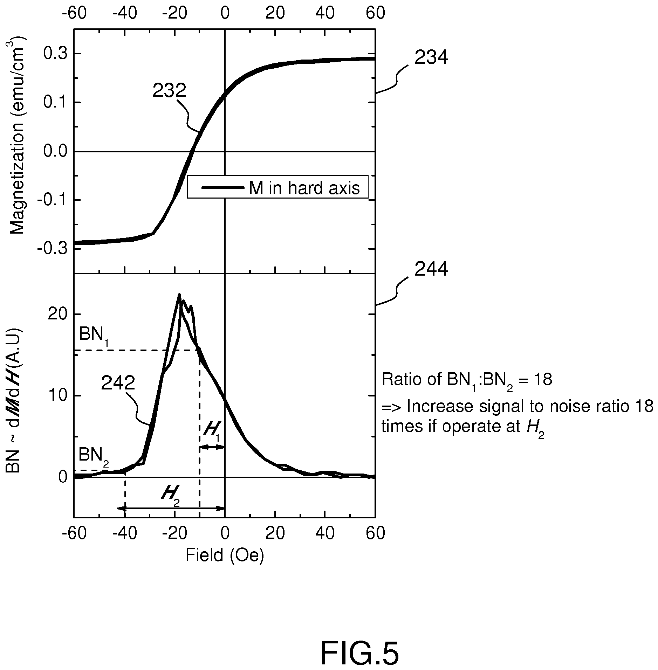

According to the Figure 5, a first curve 232 of the evolution M(H) of the magnetization M of the tested magnetic sensor 12 versus the applied constant field H is shown in the top view 234, while a second curve 242 of the evolution of the derivation dM/dH(H) versus the applied constant field H is shown in the bottom view 244. The derivative dM/dH($H_1$) at $H_1$ is about 18 times higher than the derivative dM/dH($H_2$) at $H_2$ as shown on the second curve 242. Thus, the Barkhausen noise at $H_2$ is smaller than at $H_1$, which results in a higher SU/N ratio when detecting the magnetic particles 28. To measure Barkhausen noise of the sensor 12, the inventors applied an AC magnetic field 5 Oe$_{rms}$ at frequency of 113.7 Hz, and recorded voltage variations are at two bias DC magnetic fields $H_1$ and $H_2$. The root-means-square of the voltage noise ($\Delta V_{Barkhausen}$) are measured to be about 309.8 nV and 60.07 nV respectively. These results reveal that the Barkhausen noise is about three orders of magnitude higher than the voltage noise caused by thermal effect (Thermal noise). Therefore, Barkhausen noise is dominating to be the main noise source in the investigated MR biosensor 12.

Finally, taking into account that the same voltage change ΔV is involved by the magnetic particles 28 at $H_1$ and $H_2$ for the tested sensor, the ratio Su/N at $H_2$ is found 5.15 times higher than the ratio Su/N at $H_1$. Thus selecting the amplitude of the magnetic field applied by the magnetic coil 18 to be comprised in the vicinity of $H_2$, namely comprised between 40 Oe and 60 Oe, allows achieving a high sensitivity.

When selecting the local optimum amplitude H2 of the permanent DC magnetic field closest to the actual amplitude of the applied DC constant magnetic field among the two optimum amplitudes H1, H2 of the set as the local optimum amplitude that maximizes the second quantity $H \cdot \dfrac{\partial V_{MR}}{\partial H} / BN(H)$ the limit or the threshold of detection (LOD) of the particle attainable by the measurement system 2 is lowered.

Turning to the Figure 1, the signal processing device 20 is adapted to deduce at least one physical quantity based on the differential voltage measured by the voltage measurement device 16.

The signal processing device 20 is further configured to deduce at least one physical quantity based on the extracted amplitude.

The physical quantity is relative to the at least one single magnetic particle 28.

Especially, the physical quantity is relative to the number of magnetic particles 28 bound closely to the magnetic track 24 through sandwich-type aptamer structures 27, hence the concentration of the specific bio-particles of the solution.

Especially the multi-ring geometry of the magnetic track 24 and the connectivity of the four arms lead to a Wheastone bridge configuration.

Generally, the differential output voltage V$_{MR}$ of the magnetic sensor 12 is given by the equation:

$$V_{MR} = V_{stray} + V_{drift} = \langle H_{stray} \rangle S(H) + I.R_{offset}$$

where

$V_{stray}$ the voltage response horizontally caused by the stray field generated from the magnetic particles bound close to the magnetic track 24,
I is the DC bias current injected in the magnetic track 24 of the sensor 12,
$\langle H_{stray} \rangle$ is the stray field from the magnetic particles averaged over the magnetic sensor active surface, and
S(H) is the sensitivity of the magnetic sensor at the applied constant magnetic field.

The single ring or multi-ring architecture advantageously provides with high sensing performance due to the Wheastone bridge electrical configuration.

Here, the signal processing device 20 comprises a processor 48 but does not include a lock-in unit.

The processor 48 is configured to process the amplitude of the magnetic measured signal $V_{MR}$ and to determine at least one physical quantity based on the differential voltage $V_{MR}$ and representative of the bio-molecules concentration.

Notably, the processor 48 is configured for detecting and quantifying from a set of different differential voltages $V_{MR}$ (t), measured by the voltage measurement device 16 and sampled at different time t, a magnetic flux change representative of the kinetics of the binding of the magnetic particle 28 close to the active surface 22 of the sensor 12 through the binding to the sandwich-type aptamer structures 27.

Optionally the measurement system may further include an environmental temperature sensor 50 and a temperature controller 52.

The environmental temperature sensor 50 is configured to measure a temperature representative of the actual environmental temperature T of the magnetic particles 28 and the sensor 12, preferably located close to the active surface 22 of the sensor 12. For instance, the environmental temperature sensor 16 is a platinum resistance of 100 Ohms.

The temperature controller 52 is adapted to control and/or to regulate the environmental temperature of the magnetic particles 28. More specifically, the temperature controller is configured to heat by a heater 54 in a controlled way the environment of the active surface 22 and the magnetic particles 28.

The heater 54 is for instance a Peltier heat sink element attached to the first magnetic sensor 4 and connected to a temperature controller 56 that can control the environmental temperature T variation by varying the temperature T from 77 K to 690 K.

Owing to the value of the constant magnetic field applied, the sensitivity of the measurement system 2 is improved, thus allowing the detection of a very small number of magnetic particles 28 on the magnetic track 24.

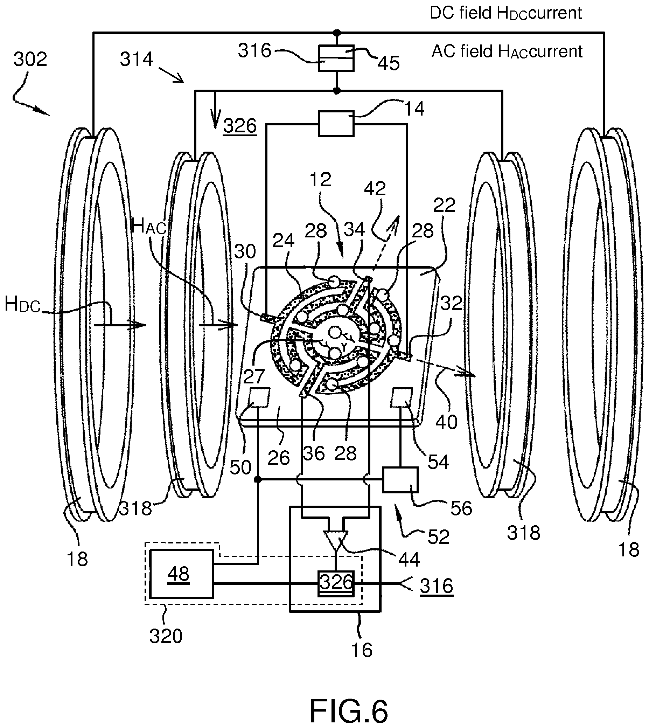

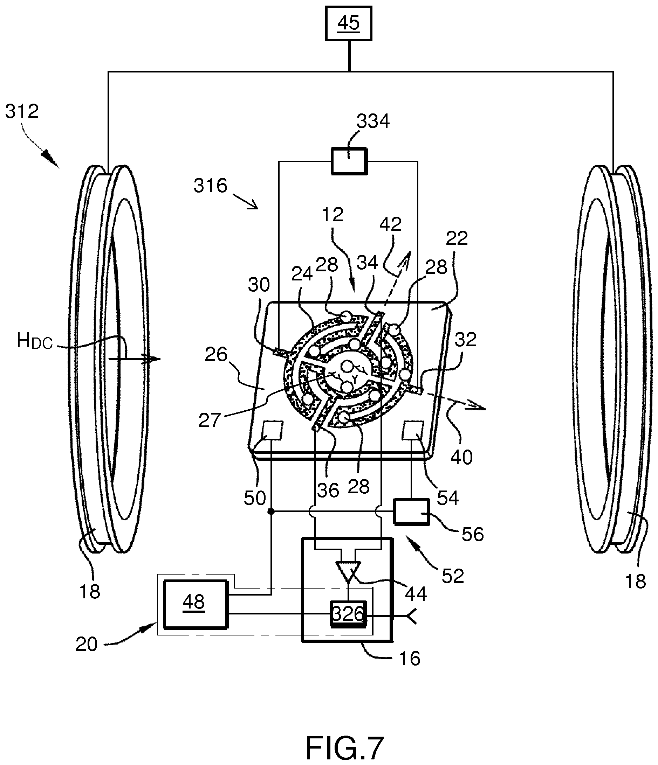

According to the Figures 6 and 7 and two variants of a same second embodiment of the invention, respective magnetic measurement systems 302, 312 are derived from the magnetic measurement by sharing common elements, in particular the aptamer-based magnetic hybrid AMR/PHR sensor 12 and the magnetic element 18, configured to apply the applied DC constant magnetic field at an actual amplitude closely located in a vicinity of one of the optimum amplitudes among the two optimum values H1 and H2

The two variants 302, 312 of the second embodiment of the magnetic measurement system differs from the magnetic measurement system 2 according to the first embodiment in that they comprise further an AC magnetic field source 314, 316 for applying an actual AC alternative magnetic field on the magnetic track 24 at a predetermined amplitude and at a predetermined frequency, the applied actual AC alternative magnetic field being collinear and added to the actual constant DC magnetic field applied by the magnetic element.

The predetermined frequency of the applied AC alternative magnetic field is ranging from 10 Hz to 3 kHz.

According to the two variants, the actual applied magnetic field H applied at the magnetic track 24 of the sensor 12 is a sum of the actual applied constant magnetic field $H_{DC}$ and an actual alternative magnetic field $H_{AC}$ according to the equation:

$$H = H_{DC} + H_{AC}$$

Advantageously, the application of an alternative magnetic field $H_{AC}$ superimposed to the constant magnetic field $H_{DC}$ allows the sensor 12 to operate at its maximum sensitivity when detecting magnetic particle bound close to the magnetic track 24, and to lock in with an alternative measured voltage signal at a suitable frequency for further minimizing the measurement noise.

According to the Figure 6 and the first variant of the second embodiment of the invention, the AC magnetic field source 314 of the magnetic measurement system 302 is an external AC magnetic source that comprises here a second AC coil current source 316 supplying a AC current oscillating at the frequency of 100 Hz, and at least one coil, here two Helmholtz coils 318, connected to the second AC coil current source 316.

The magnetic measurement system 302 differs from the magnetic measurement system 2 of the Figure 1 in that the signal processing device 20 is replaced by a signal processing device 320 that comprises further to the processor 48 a lock-in unit 326.

The lock-in unit 326 is configured to lock the amplified measured differential voltage signal onto the magnetic alternating excitation field signal by using a phase lock loop and to detect the peak measured differential voltage measured at the output of the pair of voltage terminals 34, 36.

In a variant, the lock-in unit 46 is configured to extract the amplitude at twice the exciting frequency in the differential voltage measured by the voltage measurement device 16.

The processor 48 is configured to process the amplitude extracted by the lock-in unit 46 and to determine at least one physical quantity based on the extracted amplitude, representative of the quantity of the magnetic particles 28 bound close to the active surface 22 of the sensor 12 at the time of measurement and representative of the bio-particles concentration when the saturation time of the binding of the magnetic particles has been reached.

Notably, the processor 48 is configured for detecting and quantifying from a set of the peak amplitudes Vp (t) of the measured differential voltages extracted by the lock-in unit 326 and sampled at different time t, a magnetic flux change representative of the kinetics of the binding of the magnetic particles 28 close to the active surface 22 of the sensor 12 through the binding to the sandwich-type aptamer structures 27.

According to the Figure 7 and the second variant of the second embodiment of the invention, the AC magnetic field source 316 of the magnetic measurement system 312 comprises an electrical AC bias current source 334 and the magnetic track 24.

The electrical AC bias current source 334 replaces here the electrical DC bias current source 14 of the magnetic measurement systems 2, 302 of the Figures 1 and 6, and is configured to inject an alternative AC bias current in the magnetic track 24 at a constant predetermined frequency ranging from 5 Hz to 10 kHz, here set ot 100 Hz.

By self-induction, the magnetic track 24 of the first magnetic 12 supplied with an alternating bias current AC by the AC bias current source 334 generates a magnetic excitation field $H_{AC}$ that modulates the actual constant DC magnetic field $H_{DC}$ applied by the magnetic element 18 at an actual amplitude closely located in a vicinity of one of the two optimum amplitudes H1 and H2.

The intensity of the alternating bias current is chosen high enough so that the range of the amplitude of the total magnetic excitation field H actually applied includes a single optimum value among H1 et H2, and is chosen low enough so that the total magnetic excitation field H actually applied is kept closest to the optimum value H1 or H2.

The electrical AC bias current source 334 is for example a Keithley 6221 AC Current source or Agilent 33522A voltage source and the amplitude of the AC bias current being set equal to 1 $mA_{rms}$, the term "rms" standing for "root means square".

The processor 48 is configured in the same way as described for the magnetic measurement system 302 of the first variant.

Regardless the considered first embodiment and the two variants of the second embodiment, the measurement magnetic system comprises in variants one or several of the following features:

- the magnetic track is chosen in the group consisting of a cross track, a ring track and a multi-ring track;
- the magnetic track is a ring-track, the number of rings being comprised between 1 and 17;
- the magnetic track is made in a structure chosen in the group consisting of a tri-layered structure including a ferromagnetic film, a metal and an anti-ferromagnetic film, in particular Ta/NiFe/Cu/IrMn/Ta, a bilayer structure, in particular Ta/NiFe/IrMn/Ta, and a spin-valve, in particular Ta/NiFe/Cu/NiFe/IrMn/Ta.

In another variant, the magnetic measurement system comprises two AMR/PHR magnetic sensors having a same magnetic structure and a same shape amongst the cross shape, the single ring closed loop shape, or the multi-ring with four arms shape.

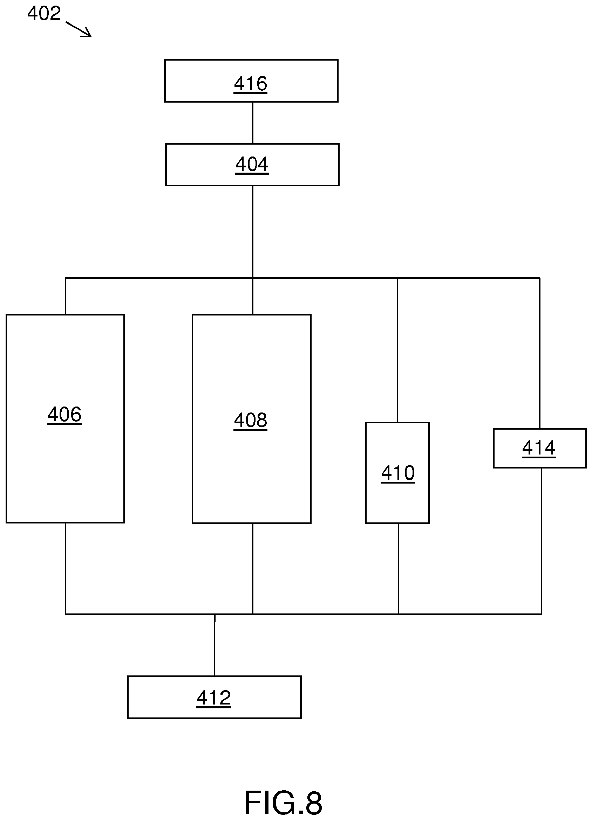

According to the Figure 8, a magnetic measuring method 402 for measuring low concentrations of bio-particles contained in a given solution and/or quantifying the interactions of specific bio-particles contained in a solute at an unknown concentration, in particular for concentrations ranging from nano-moles to pico-moles comprises several steps.

In a first step 404 a magnetic measurement system, configured for measuring concentration of bio-particles through a sensitive detection of magnetic particle labels, is provided, as for example the magnetic measurement systems 2, 302, 312 respectively described in the Figures 1, 6 and 7.

Generally the magnetic measurement system comprises:

- an aptamer-based magnetic hybrid AMR/PHR sensor 12 including :

  • a prepared active surface 22 on which at least one magnetic particle 28 is ready to be or is closely bound through a sandwich-type aptamer structure 27 that includes a captured unitary bio-particle, the active surface 22 including a magnetic track 24 with a closed loop shape,
  • a first current terminal 30 and a second terminal 32, forming a pair of current terminals 30, 32 facing each other and contacting with the closed loop magnetic track 24,
  • a first voltage terminal 34 and a second voltage terminal 36 forming a pair of voltage terminals facing each other and contacting with the closed loop magnetic track 24.

Then, in a second step 406, an electrical bias current is injected permanently in the magnetic track 24 by using the electrical bias current source 14 connected between the first and second current terminals 30, 32.

In parallel to the second step 406 and in a third step 408, an actual DC constant magnetic field is applied permanently on the magnetic track 24 at predetermined amplitude by using a magnetic element 18.

After the start of second and third steps 406, 408 and in a fourth step 410, while the injection of the electrical bias current in the magnetic track 24 and the application of the actual DC constant magnetic field still continue, a differential voltage $V_{MR}$ between the pair of voltage terminals 34, 36 is measured by a voltage measurement device 16 connected between the first and second voltage terminals 34, 36.

Then in a fifth step 412, at least one physical quantity is determined by the signal processing device 20 on the basis of the measured differential voltage, the physical quantity being representative of the bio-particles concentration.

The magnetic measurement method 402 is characterized in that the actual amplitude of the applied DC constant magnetic field is closely located in a vicinity of an optimum amplitude of the permanent DC magnetic field comprised in a set of two optimum values H1, H2 of the permanent DC magnetic field, each optimum value H1, H2 maximizing locally the absolute value of a first quantity $H \cdot \dfrac{\partial V_{MR}}{\partial H}$, where H is the amplitude of the applied magnetic field and $\dfrac{\partial V_{MR}}{\partial H}$ is the derivate of the differential voltage with respect to the magnetic field at the applied field H.

The magnetic measurement method is also characterized in that the said method comprises a sixth step 414 of applying on the prepared active surface 22 a drop of a magnetic solution containing streptavidin-coated magnetic nanoparticles at a saturated concentration in regard of the concentration of the specific bio-molecules to be determined.

When only the concentration of the specific bio-molecules is measured, the sixth step is preferably applied before the measurement step, i.e the fourth step 410, the measurement step 414 being started once the saturation time of the binding of the magnetic particles to the sandwich a sandwich-type aptamer structures 27 being attained.

When quantifying of the interactions of the specific bio-particles is sought and as shown in Figure 8, the sixth step 414 is executed after the start of the second and third steps 410, 412 of injecting a bias current in the magnetic track and applying the actual constant magnetic field and after starting the fourth step of measuring the differential voltage $V_{MR}$ until at least the saturation time of the binding of the magnetic particles to the sandwich-type aptamer structures 27 is achieved.

Preferably, the local optimum amplitude H2 of the permanent DC magnetic field closest to the actual amplitude of the applied DC constant magnetic field is selected among the two optimum amplitudes H1, H2 as the local optimum amplitude that maximizes a second quantity $H \cdot \dfrac{\partial V_{MR}}{\partial H} / BN(H)$ defined as the ratio of the first quantity $H \cdot \dfrac{\partial V_{MR}}{\partial H}$ to the Barkhausen noise level BN(H) at the applied field, the Barkhausen noise being proportional to $\dfrac{dM}{\partial H}(H))$, where M is the magnetization of the magnetic sensor.

The magnetic measuring method 402 comprises further a seventh step 416 of preparing the active surface 22 by:

- modifying the active surface with thiol-labeled primary aptamer, then
- applying a drop of a solution of specific bio-particles at a concentration to be determined to the primary immobilized surface, the volume of the drop being a priori known, then
- using biotin-labeled secondary aptamers to bind with the bio-particles.

The seventh step 416 is executed before the first step 404 of providing a magnetic measurement system according to the invention.

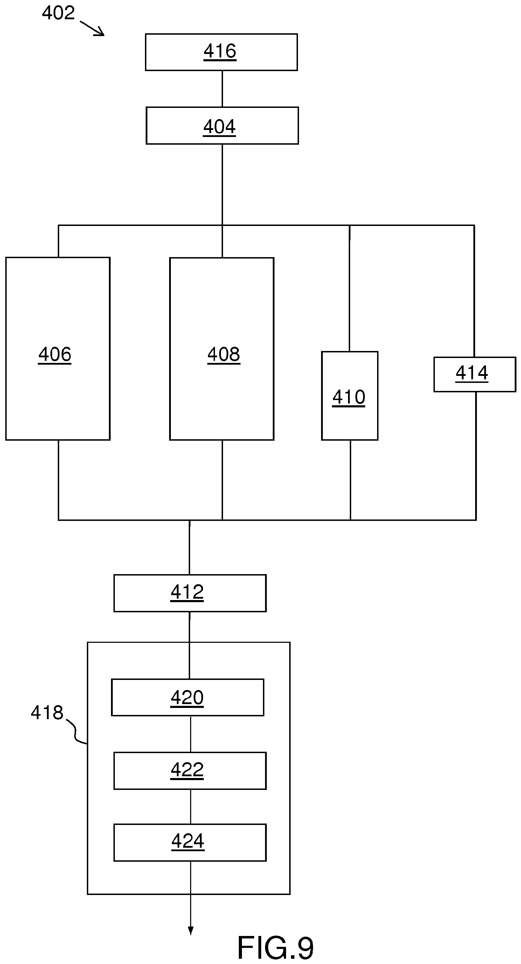

According to the Figure 9 and when the magnetic measurement method 402 is aimed to quantify the interactions of specific bio-particles contained in a solution at an unknown concentration, the said magnetic measurement method 402 comprises a supplemental signal processing step, forming a eighth step 418 and executed after the fifth step.

The eighth step 418 includes a ninth step 420, a tenth step 422 and an eleventh step 424, executed sequentially.

In the ninth step 420 the kinetics of the binding of the magnetic particles to the specific bio-particles captured and immobilized on the active surface is determined from the temporal evolution of the measured magnetic signal.

In the tenth step 422, a saturation time from the binding kinetics of the magnetic particles is determined.

In the eleventh step 424, from the saturation time and a model function an average distance separating the bio-particles in their solution is computed and the interactions of specific bio-particles are quantified according a breakdown of the forces distinguishing the contribution of the long-range Coulomb interaction and the short-range Van de Waals interaction.

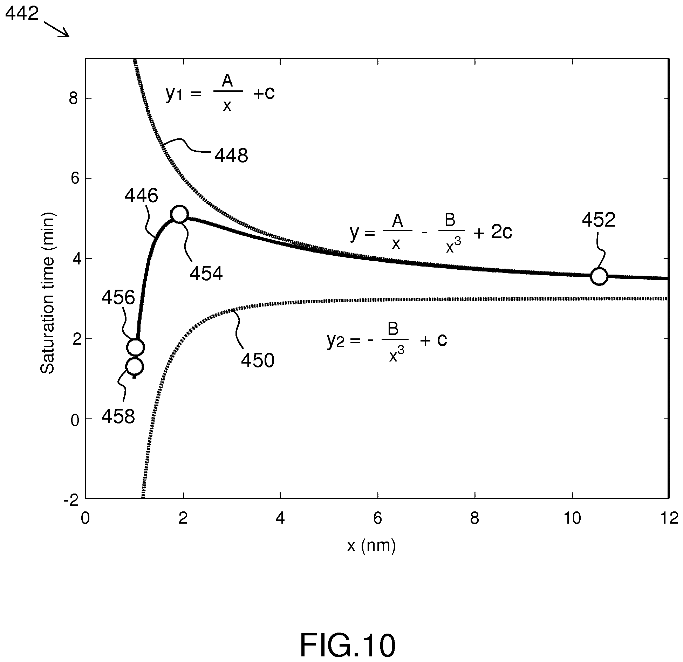

According to Figure 10, a model 442 for quantifying the interactions of specific bio-particles as thrombin for example comprises a first curve 446, a second curve 448 and a third curve 450.

The first curve 446 is a first model function that shows the evolution of the saturation time expressed in minute(s), as well as the total interaction force between two specific neighboring immobilized bio-particles, here thrombin molecules, versus the distance x, expressed in nm, separating the two specific neighboring immobilized bio-particles. This first curve can be analytically expressed by:

$$y = \frac{A}{x} - \frac{B}{x^3} + 2C$$

where A, B, C are three fitting parameters to be determined.

The second curve 448 is a second model function that represents the contribution of the attractive force due to Coulomb interaction to the total interaction force between the two specific neighboring immobilized bio-particles, here thrombin molecules, versus the distance x, expressed in nm, separating the two specific neighboring immobilized bio-particles. This second curve can be analytically expressed by:

$$y_1 = \frac{A}{x} + C$$

where A and C are the same fitting parameters as defined for the first curve 446.

The third curve 450 is a third model function that represents the contribution of the repulsive force due to Van-der-Waals interaction to the total interaction force between the two specific neighboring immobilized bio-particles, here thrombin molecules, versus the distance x, expressed in nm, separating the two specific neighboring immobilized bio-particles. This third curve 450 can be analytically expressed by:

$$y_2 = -\frac{B}{x^3} + C$$

where B and C are the same fitting parameters as defined for the first curve 446.

The first curve 446 is the sum of the second curve 448 and the third curve 450 as expressed by the equation:

$$y = y_1 + y_2$$

The fitting parameters A, B and C are determined by fitting the first curve 446 with several measurements of saturation times corresponding to different concentrations of the bio-particles, here thrombin, immobilized to the sensor surface through the aptamer sandwich structure, and defining here four points 452, 454, 456, 458 of the first curve 446.

The first, second, third points 452, 454, 456 illustrate the measured saturation times corresponding to the respective concentrations of thrombin equal to 86 pM, 0,86 nM and 8,6 nM. The fourth point 458 provides with a same value of the measured saturation time corresponding to the thrombin concentration.

Here, the fitting parameters A, B and C are equal to 6, 8 and 3.

TEST AND PERFORMANCE RESULTS

Magnetic measurement performance

The improvement of the minimum detection level of the magnetic measurement system 2, 302, 312 according to the invention has been measured and confirmed by experiments using streptavidin-biotin hybridization without embedding here a specific bio-particle in the sandwich aptamer structure.

The voltage changes of the biosensor according to the invention caused by magnetic particles bound to the magnetic track 24 have been measured and compared by applying a constant magnetic field H1 or a constant magnetic field $H_2$.

For the manufacture of any of the sensors 12, 2-inch sputtering targets of $Ni_{80}Fe_{20}$ (99.99%), Ta (99.999%), $Ir_{25}Mn_{75}$ (99.9%), Cu (99.999%), Au (99.999%) (Kojundo Chemical Laboratory, Japan) and $SiO_2$ (99.99%, Wafermart, Korea) were used to fabricate sensor stack, electrode and passivation layer. Photoresist AZ 5214E and Developer AZ 500MIF (AZ Electronic Materials, USA) were used in photolithography process. Epoxy (PT-135K, Poly-tech Co. Ltd., Korea) was use for protecting the wire bonding contact from the sensor electrode to printed circuit board.

More specifically, each sensor 12 was fabricated on $Si/SiO_2$ (1000 nm) substrates using a standard UV photolithography and lift-off methods. Sensor material is a tri-layer stack Ta/NiFe/Cu/IrMn/Ta (5/10/0.1/10/5 nm) fabricated using a DC magnetron sputtering (Seoul vacuum, Korea). The sensor shape is a cross-juntion of 50 μm × 50 μm. The lithography and lift-off processes are repeated the same for the fabrication of electrode Ta/Au (5/50 nm), passivation layer SiO$_2$ (200 nm) and activation layer Ta/Au (5/10 nm). The sensor was connected to a customized printed circuit board (PCB) using a wire bonding system (West Bond 7327C, USA) and packaged using UV epoxy with open active surface for measurement of magnetic particles.

Immobilization of the sensors surface with biotin was done with the following protocol. Especially, the sensor surface of SiO2 is modified by APTES (an aminosilane) and then functionalized with biotin.

The modification of the sensor surface by APTES was performed with the following protocol:

1. Clean sensor surface by DI water and dry by N2 blow (Keep the sensor surface at room temperature)
2. Keep at 70°C for 10 min, and wait till the sensor cool down at room temperature.
3. Make APTES solution in DMSO (5 % w/v) (at room temperature, if it freeze then keep the heater for the solution, noted that the tip also should be the same temperature with the solution).
4. Put 1 ml on the sensor surface (make sure there is no precipitation spot of APTES on the sensor surface)
5. Keep 4 hours at humidity chamber at 90 %, room temperature and normal pressure. (Check the precipitation after 1 hour)
6. Wash by DI water, 200 ml, 2 - 3 times to remove unbound APTES
7. N2 blow for drying the sample and store at room temperature.

The functionalization with biotin was performed using the following protocol:

1. Chromalink Biotin desolve in DMSO (5 mg/500 ml) -> solution A
2. TEA in DMSO 2 % (volume/volume) -> solution B
3. 10 ml solution A in 5 ml solution B (mixture by mixer) -> solution C
4. Use 4 ml solution C on APTES modified sensor surface
5. Store in inert Ar atmosphere for 4 hours
6. Wash 100 ml x 3 DI water (3 times)
7. Store at 4 - 8 degree, refrigerator.

Streptavidin functionalized magnetic beads SiMAG-Streptavidin (Chemicell GmbH, Germany) with the mean size of 100 nm were used as magnetic particles.

Especially, magnetic nanoparticles are modified and functionalized with APTES and streptavidin. Streptavidin and biotin are to demonstrate the high resolution of the sensor because they have very strong bio affinity.

The following reagents were used for the modification of the magnetic nanoparticles with APTES:

Solution A: APTES in EtOH 5 % (weight/volume)
Solution B : Amonium hydroxide 29 %
0.5 mg of Fe3O4 was diluted in 2 ml of solution A and 20 ml of solution B.

The mixture was sonicated for 4 hours at room temperature, the magnetic particles collected by using a permanent magnet, washed three times in DI water, followed by dissolution in PBS (pH = 7.4) in 5 ml.

These steps were repeated 3 times to increase the binding affinity. The magnetic nanoparticles were then stored in a refrigerator at 4 - 8 degrees for later usage.

For the immobilization of streptavidin on the magnetic nanoparticles modified with APTES, the following procedure was applied:

1. PBS + NPs -> solution A
2. Mix 10 mg EDC in 1ml solution A (used 50 mg in 5 ml solution A)
3. Steptavidin: 5 ml (stock solution) in 1 ml solution A (we have used dilution of 1:100 stock solution)
4. Add stretavidin stock solution in to the mixture PBS+NPs+EDC
5. Rotating shaker over night at 4 °C
6. Washed by DI water for 2 times x 1 ml
7. Dispersed NPs in PBS solution and store at 4 - 8 °C.

The streptavidin functionalized magnetic particles were deposited on the sensor, to bind with the biotin, with concentrations of 1.5 fM, 3 fM, 15 fM, 150 fM and 1.5 nM.

The voltage change induced by the magnetic particles was then measured for each concentration. The measurements showed that the voltage variation at H$_2$ is smaller than that at H$_1$ due to small Barkhausen noise. The S/N

ratios measured at $H_2$ and $H_1$ are corresponding to 12.5 and 2.4, confirming the inventor's finding that higher sensitivity in detection of magnetic particles when the magnetic field applied is $H_2$.

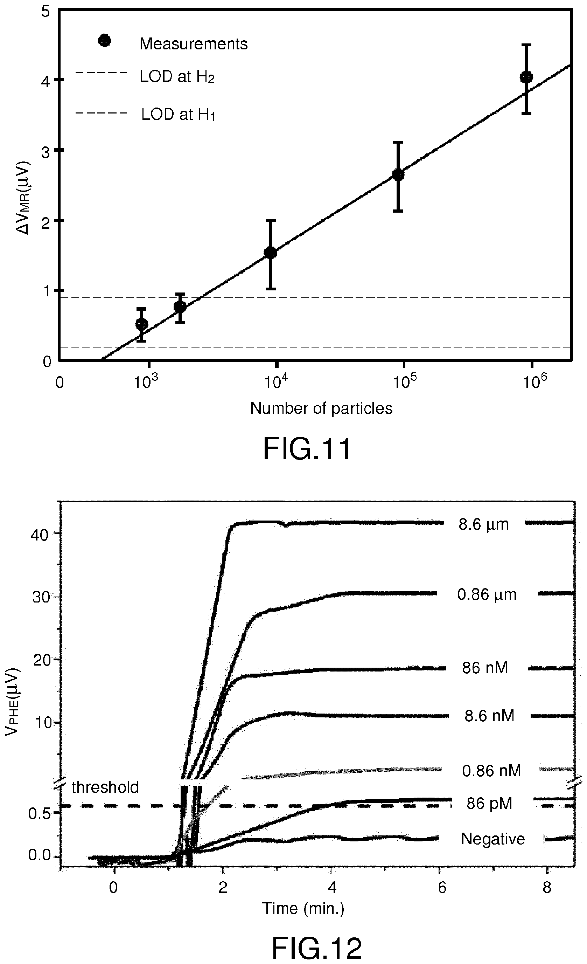

The Figure 11 illustrates the voltage changes measured as a function of the concentration of magnetic particles, and the limits of detection (LOD) for two sets of experiments at two fields $H_1$ and $H_2$. The voltage change resulting from the magnetic particles ($\Delta V_{particles}$) increases linearly with log scale of particle number. The high-accuracy of the fitting of 98 % shows that the measurement system and more specifically the magnetic sensor presents a high sensitivity and a good reproducibility for quantification of magnetic particles.

LOD at applied field $H_1$ and $H_2$ are determined from linear fitting line to be as low as 2800 and 550 particles per micro-litter corresponding to 4.6 fM and 0.9 fM respectively. Here, the LOD is calculated based on signal-to-noise approach, i.e., the signal is 3 times of standard deviation of the voltage variation recorded consecutively in 6 minutes without magnetic labels. For reproducible signals and accurate quantification, the utilized sensors have similar magnetic sensitivity and surface modification process. The magnetic sensor exhibits feasibility in detection of ultra-low quantity of magnetic labels when the constant magnetic field applied is $H_2$, down to one femto-Molar resolution. The detectable amount of the MR sensor is estimated to be 550 NPs (0.22 nanoparticle per $\mu m^2$) corresponding to the magnetic moment of $1.2 \times 10^{-13}$ emu measured in buffer solution. In this estimation, we consider the susceptibility of nanoparticles, $\chi_V = 5$, the mean radius of the particles (r = 50 nm). Magnetic moment of the detectable amount can be calculated by using equation $m = n V_{particle} \chi_V H$. This ultralow detection limit allows enhancing the sensitivity of the sensor by at least 3 orders of magnitude compared to sensors according to the prior art.

Detection of thrombin and aptamer DNA interaction

The inventors further used the highly sensitive magnetic sensor 12 to the detection of thrombin, one of the most important biomarkers in the blood.

Sensors were prepared by fabricating a tri-layer stack Ta/NiFe/Cu/IrMn/Ta (5/10/0.1/10/5 nm), as described above.

In this study, thrombin was sandwiched between two aptamers, a thiolated 15-mer DNA aptamer covalently bound to Au on the sensor surface (primary aptamer) and a biotinylated 15-mer DNA aptamer probe bound to the target thrombin with biotin (secondary aptamer).

Thiolated and biotinated 15-mer aptamers of 70 ng/$\mu$L (GeneChem Inc, Korea) and human $\alpha$-thrombin of 8.6 $\mu$M (Sigma Aldric, USA) were used.

Sequence of the probes are as following:

Thiolated 15-mer DNA aptamer probe: 5'-SH-$(CH_2)_6$-poly(14 T) GGT TGG TGT GGT T GG-3'
Biotinylated 15-mer DNA aptamer probe: 5'-biotin-GGT TGG TGT GGT TGG-3'.

Stock thiolated 15-mer aptamer was diluted in Tris-EDTA buffer to a final concentration of 70 ng/$\mu$L. A volume of 5 $\mu$L was spotted on the clean Au surface of sensors. The reaction was kept overnight in open air to activate the immobilization of thiol group on Au surface. The process was followed by washing 3 times in Tris-EDTA buffer to remove the unbound aptamers. Then, each droplet 2$\mu$L of human thrombin at different concentration (8.6 $\mu$M to 86 pM with a 10x dilution) was addressed to 6 modified sensor surfaces and then incubating them in 1h at 37 °C to activate thrombin - aptamer binding. After that, the samples were washed 3 times in PBS buffer to remove unbound thrombin. Finally, a spot 5 $\mu$L of 70 ng/$\mu$L biotinylated 15-mer aptamer solution was added to accomplish the sandwich structure by allowing incubation in 1h at 37 °C. The sensors were then washed again with Tris-EDTA buffer and stored at 4 °C.

2 $V_{DC}$ bias voltage was applied to the sensors along the easy axis of magnetic anisotropy of sensor using an Agilent function generator (Agilent 33522A, USA). Alternative (AC) magnetic field at 113 Hz from a pair of Helmholtz coils (BH175-HF Serviciencia, Spain) was applied perpendicular to the easy axis of the sensor. Frequency of 113 Hz is chosen to minimize the 1/f noise of the sensor. This frequency is higher than 1/f knee value determined from the noise measurement. A constant magnetic field at an amplitude $H_2$ was further applied.

The voltage signals of the sensors were detected by means of a lock-in amplifier (RS830, Stanford research, USA) using first harmonic and 30 data points averaging setup. Measurement setup including electronics devices and sensor circuits are placed inside a homemade Faraday cage to minimize electrical noises of the measurement system.

The Figure 12 illustrates real-time voltage measurements of different thrombin concentration modified on sensor surfaces in response to the same quantity of magnetic labels. Positive samples are the measurement of sensor with modified thrombin concentration from 8.6 $\mu$M to 86 pM with 10-fold dilution. Negative sample is the measurement of non modified sensor. Detection threshold of MR sensor is calculated by three times voltage signal of the non modified sensor with respect to the spot of magnetic labels (negative sample). These measurements clarified two fundamental information concerning a biochip: LOD of thrombin concentration and detection threshold time with respect to thrombin concentration.

The Limit of Detection (LOD) of thrombin concentration can be calculated from Figure 12, where the changes of voltages ($\Delta V_{MR}$) are plotted with thrombin concentration. LOD of thrombin concentration is calculated by the interception of $\Delta V_{MR}$ fitting line and LOD of voltage to be about 70 pM. The sensitivity of present MR sensor is thus proved to be superior in comparison with other measurement techniques.

Modeling interaction mechanism of labeled protein on a solid surface

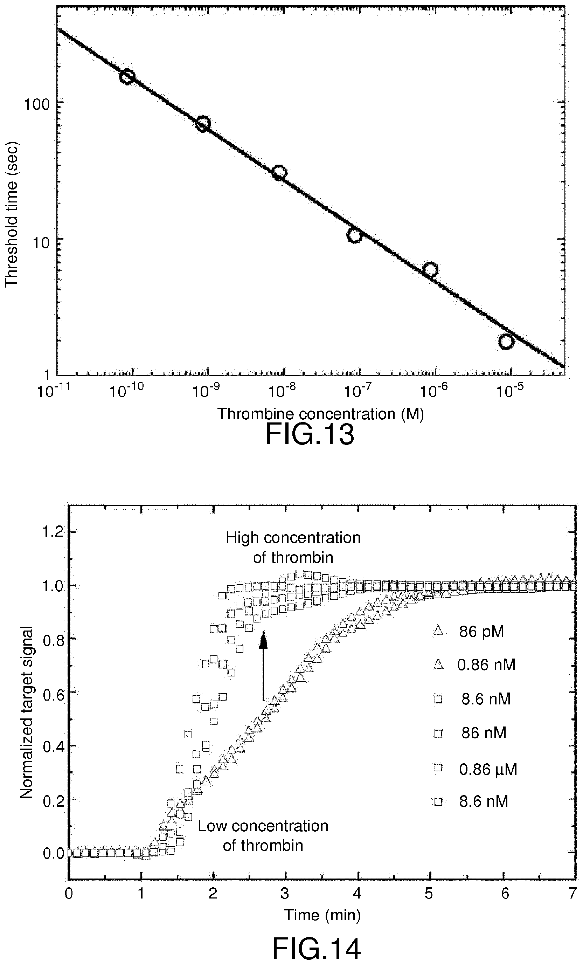

Furthermore, the inventors have investigated the variation of the detection threshold time with the thrombin concentration, which appeared to be a decreasing function of this concentration, as illustrated on the plot of Figure 13.

The plot of Figure 14, which illustrates the normalized voltage profile of the lowest and the highest concentrations of thrombin, indicates that steric hindrance and electrostatic repulsion effects are involved in the protein interaction.

The results of Figure 14 reveal that the slope of the binding curve is increasing with the concentration of thrombin concentration. In this case steric hindrance and electrostatic repulsion effects are not sufficiency. This is assigned for the identical case of low concentration of thrombin (minimize steric hindrance effect) and highly affinity of aptamer and thrombin interaction resulting in less efficiencies of electrostatic repulsion effect. The binding efficiency in this finding is opposite for the binding efficiency of conventional DNA interaction where the steric hindrance and electrostatic repulsion effect are strong. Wherein, the slope of binding curve is decreasing with concentration of DNA concentration.

Theses result show that the measurement system and method according to the invention can be used with profit to study interaction principle such as attractive and repulsive force of biomarkers using saturation time of the measured signals as already described here above in the Figures.

The measurement system according to the invention therefore opens up possible studies of phenomena which could not be investigated until now.

**Claims**

1. A magnetic measurement system for measuring a concentration of specific bio-particles through a sensitive detection of magnetic particle labels comprising:

   - an aptamer-based magnetic hybrid Anisotropic Magnetoresistive/ Planar Hall Magnetoresistive (AMR/PHR) sensor (12) having :

     • an active surface (22) on which at least one magnetic particle (28) is ready to be or is closely bound through a sandwich-type aptamer structure (27) that includes a captured unitary bio-particle, the active surface including a magnetic track (24) with a closed loop shape,
     • a first current terminal (30) and a second terminal (32), forming a pair of current terminals (30, 32) facing each other and contacting with the closed loop magnetic track (24),
     • a first voltage terminal (34) and a second voltage terminal (36) forming a pair of voltage terminals facing each other and contacting with the closed loop magnetic track (24),

   - an electrical bias current source (14) configured to inject a bias current in the magnetic track (24), the electrical source (14) being connected between the first and second current terminals (30, 32), and
   - a voltage measurement device (16) connected between the first and second voltage terminals (34, 36) measuring a differential voltage $V_{MR}$ between the pair of voltage terminals (34, 36),
   - a magnetic element (18) for applying an actual DC constant magnetic field on the magnetic track at a predetermined amplitude;
   - a signal processing device (20; 320) configured to deduce at least one physical quantity based on the differential voltage and representative of the bio-particles concentration

   **characterized in that**
   the magnetic measurement system is being configured to choose
   the actual amplitude of the applied DC constant magnetic field to be closely located in a vicinity (218, 210) of an optimum amplitude of the permanent DC magnetic field comprised in a set of two optimum values H1, H2 of the permanent DC magnetic field, each optimum value H1, H2 maximizing locally the absolute value of a first quantity

$$H \cdot \frac{\partial V_{MR}}{\partial H},$$

where H is the amplitude of the applied magnetic field and $\frac{\partial V_{MR}}{\partial H}$ is the derivate of the differential voltage with respect to the magnetic field at the applied field H.

2. The magnetic measurement system according to claim 1, wherein
the diameter of the vicinity (218, 210) surrounding the optimum amplitude H1 and H2, of the permanent DC magnetic field is lower than a first predetermined threshold so that a first ratio of the absolute value of the first quantity

$$H \cdot \frac{\partial V_{MR}}{\partial H}$$

at the actual amplitude of the applied DC constant magnetic field over the maximum absolute value of the quantity $H \cdot \dfrac{\partial V_{MR}}{\partial H}$ at the corresponding optimum amplitude H1 or H2 is lower than minus 3 dB.

3. The measurement system according to any of claims 1 to 2, wherein the local optimum amplitude H2 of the permanent DC magnetic field closest to the actual amplitude of the applied DC constant magnetic field is selected among the two optimum amplitudes H1, H2 of the set as the local optimum amplitude that maximizes a second quantity

$$H \cdot \frac{\partial V_{MR}}{\partial H} / BN(H)$$

defined as the ratio of the first quantity $H \cdot \dfrac{\partial V_{MR}}{\partial H}$ to the Barkhausen noise level BN(H) at the applied field, the Barkhausen noise being proportional to $\dfrac{dM}{\partial H}(H))$, where M is the magnetization of the magnetic sensor.

4. The magnetic measurement system according to any of claims 1 to 3, wherein
the diameter of the vicinity surrounding the optimum amplitude H1 or H2 of the permanent DC magnetic field is lower than a second predetermined threshold so that a second ratio of the absolute value of a second quantity

$$H \cdot \frac{\partial V_{MR}}{\partial H} / BN(H)$$

at the actual amplitude of the applied DC constant magnetic field over the maximum absolute value of the second quantity $H \cdot \dfrac{\partial V_{MR}}{\partial H} / BN(H)$ at the corresponding optimum amplitude H1 or H2 is lower than minus 3 dB, the second quantity $H \cdot \dfrac{\partial V_{MR}}{\partial H} / BN(H)$ being defined as the ratio of the first quantity

$H \cdot \dfrac{\partial V_{MR}}{\partial H}$ to the Barkhausen noise level BN(H) at the applied field, the Barkhausen noise being proportional to

$\dfrac{dM}{\partial H}(H))$, where M is the magnetization of the magnetic sensor.

5. The magnetic measurement system according to any of claims 1 to 4,
comprising further an AC field magnetic source (314; 316) for applying an actual AC alternative magnetic field on the magnetic track (24) at a predetermined amplitude and at a predetermined frequency, the applied actual AC alternative magnetic field being collinear to the actual constant DC magnetic field applied by the magnetic element (18).

6. The magnetic measurement system according to claim 5, wherein the AC field magnetic source (314; 316) is either an external magnetic field source including at least one coil (318) and a coil current source (317) for supplying an AC current source, or is
an electrical AC bias current source (334), configured to inject an alternative AC bias current in the magnetic track (24), the magnetic track (24) generating accordingly by self induction the applied AC field.

7. The magnetic measurement system according to any of claims 1 to 6, wherein the magnetic element (18) is a set comprising at least one magnetic coil or a permanent magnet.

8. The magnetic measurement system according to any one of claims 1 to 7, wherein the magnetic track (24) is chosen in the group consisting of a cross track, a ring track and a multi-ring track.

9. The magnetic measurement system according to any of claims 1 to 8, wherein the magnetic track (24) is a ring-track, the number of rings being comprised between 1 and 17.

10. The magnetic measurement system according to claim 1 to 9, wherein the magnetic track (24) is made in a structure chosen in the group consisting of a tri-layered structure including a ferromagnetic film, a metal and an anti-ferromagnetic film, in particular Ta/NiFe/Cu/IrMn/Ta, a bilayer structure, in particular Ta/NiFe/IrMn/Ta, and a spin-valve, in particular Ta/NiFe/Cu/NiFe/IrMn/Ta.

11. The magnetic measurement system according to any one of claims 1 to 10, wherein
the signal processing device (20; 320) comprises a standalone processor (48) when no AC magnetic field is applied onto the magnetic track (24), and comprises a processor (48) and a lock-in unit (326) when an AC magnetic field is applied onto the magnetic track (24); and
the signal processing device (320) is configured to extract from the differential voltage $V_{MR}$ the first or second harmonic of the differential voltage when a an AC magnetic field is applied onto the magnetic track (24); and
the signal processing (320) is configured to determine said at least one physical quantity based on said first or second harmonic, when the electrical bias current source (14) injects an alternative AC bias current in the magnetic track (24) or when an external AC magnetic fied is applied collinearly with the external constant DC magnetic field.

12. A magnetic measuring method for measuring low concentrations of specific bio-particles contained in a given solution and/or quantifying the interactions of specific bio-particles contained in a given solution, in particular in solutions having concentrations ranging from nano-moles to pico-moles,
the magnetic measuring method comprising the steps of :

- providing (404) a magnetic measurement system (2) configured for measuring a concentration of bio-particles through a sensitive detection of magnetic particle labels, the magnetic measurement system (2) comprising:
- an aptamer-based magnetic hybrid AMR/PHR sensor (12) including :

• a prepared active surface (22) on which at least one magnetic particle (28) is ready to be or is closely bound through a sandwich-type aptamer structure that includes a captured unitary bio-particle, the active surface including a magnetic track (24) with a closed loop shape,
• a first current terminal (30) and a second terminal (32), forming a pair of current terminals (30, 32) facing each other and contacting with the closed loop magnetic track (24),
• a first voltage terminal (34) and a second voltage terminal (36) forming a pair of voltage terminals facing each other and contacting with the closed loop magnetic track (24),

- injecting (406) a bias current in the magnetic track (24) by using the electrical source (14) being an electrical bias current source (14) connected between the first and second current terminals (30, 32), and
- applying (408) an actual DC constant magnetic field on the magnetic track (24) at a predetermined amplitude by using a magnetic element (18);
- measuring (410) a differential voltage $V_{MR}$ between the pair of voltage terminals (34, 36) by using a voltage measurement device (16) connected between the first and second voltage terminals (34, 36),
- determining (412) at least one physical quantity based on the differential voltage and representative of the bio-particles concentration by using a signal treatment device (20) ;

the said magnetic measurement method being **characterized in that**
the actual amplitude of the applied DC constant magnetic field is closely located in a vicinity of an optimum amplitude of the permanent DC magnetic field comprised in a set of two optimum values H1, H2 of the permanent DC magnetic field, each optimum value H1, H2 maximizing locally the absolute value of a first quantity $H \cdot \dfrac{\partial V_{MR}}{\partial H}$, where H

$$\frac{\partial V_{MR}}{\partial H}$$

is the amplitude of the applied magnetic field and $\frac{\partial V_{MR}}{\partial H}$ is the derivate of the differential voltage with respect to the magnetic field at the applied field H.

13. The magnetic measuring method according to the claim 12, comprising a step (414) of applying on the prepared active surface a drop of a magnetic solution containing streptavidin-coated magnetic nanoparticles that is executed either before the step of providing the magnetic measurement system when measuring the concentration of the bio-particles, or

after the start of the steps (406, 408) of injecting a bias current in the magnetic track (24) and applying a DC constant magnetic field onto the magnetic track and after the start of the step (410) of measuring a differential voltage $V_{MR}$ when quantifying the interactions of the specific bio-molecules.

14. The magnetic measuring method according to any of claims 12 to 13, wherein the local optimum amplitude H2 of the permanent DC magnetic field closest to the actual amplitude of the applied DC constant magnetic field is selected among the two optimum amplitudes H1, H2 of the set as the local optimum amplitude that maximizes a second

quantity $H \cdot \frac{\partial V_{MR}}{\partial H} / BN(H)$ defined as the ratio of the first quantity $H \cdot \frac{\partial V_{MR}}{\partial H}$ to the Barkhausen noise level BN(H) at the applied field, the Barkhausen noise being proportional to $\frac{dM}{\partial H}(H))$, where M is the magnetization of the magnetic sensor.

15. The magnetic measuring method according to any claims 12 to 14, comprising a step (416) of preparing the active surface (22) by

.- modifying the active surface with thiol-labeled primary aptamer, then
.- applying a drop of a solution of specific bio-particles at a concentration to be determined to the primary immobilized surface, the volume of the drop being a priori known, then
.- using biotin-labeled secondary aptamers to bind with the bio-particles.

16. The magnetic measuring method according to any claims 12 to 15, for quantifying the interactions of specific bio-particles contained in a given solution comprising a supplemental signal processing step (418) including the steps of:

.- determining (420) the kinetics of the binding of the magnetic particles to the specific bio-particles captured and immobilized on the active surface is determined from the temporal evolution of the measured magnetic signal; then
.- determining (422) a saturation time from the binding kinetics of the magnetic particles;
.- from the saturation time and a model function computing an average distance separating the bio-particles in their solution and quantifying (424) the interactions of specific bio-particles that break down into a long-range Coulomb interaction and a short-range Van de Waals interaction.

**Patentansprüche**

1. Magnetisches Messsystem zum Messen einer Konzentration von spezifischen Biopartikeln durch einen empfindlichen Nachweis von Magnetpartikelmarkierungen, umfassend:

- einen Aptamer basierten hybridmagnetischen Anisotropen Magnetoresistiven/Planaren Hall-Magnetoresistiven (AMR/PHR)-Sensor (12), der aufweist:

• eine aktive Oberfläche (22), auf der mindestens ein magnetisches Partikel (28) durch eine Aptamer-Struktur vom Sandwich-Typ (27), die ein eingefangenes unitäres Biopartikel einschließt, dicht gebunden ist oder dazu bereit ist, wobei die aktive Oberfläche eine Magnetspur (24) mit einer geschlossenen Schleifenform einschließt,

• einen ersten Stromanschluss (30) und einen zweiten Anschluss (32), die ein Paar zueinander gegenüberstehender Stromanschlüsse (30, 32) bilden und mit der Magnetspur (24) mit geschlossener Schleifenform in Kontakt stehen,
• einen ersten Spannungsanschluss (34) und einen zweiten Spannungsanschluss (36), die ein Paar zueinander gegenüberstehender Spannungsanschlüsse bilden und mit der Magnetspur (24) mit geschlossener Schleifenform in Kontakt stehen,

- eine elektrische Vorspannungsstromquelle (14), die konfiguriert ist, um einen magnetischen Vorspannungsstrom in die Magnetspur (24) einzuspeisen, wobei die elektrische Quelle (14) zwischen dem ersten und dem zweiten Stromanschluss (30, 32) angeschlossen ist, und
- eine Vorrichtung zur Spannungsmessung (16), die zwischen dem ersten und dem zweiten Spannungsanschluss (34, 36) angeschlossen ist und einen Differenzspannungs-$V_{MR}$ zwischen dem Paar von Spannungsanschlüssen (34, 36) misst;
- ein Magnetelement (18) zum Anlegen eines tatsächlichen konstanten Gleichstrom-Magnetfelds an die Magnetspur mit einer vorbestimmten Amplitude;
- eine Vorrichtung zur Signalverarbeitung (20; 320), die konfiguriert ist, um mindestens eine physikalische Größe basierend auf der Differenzspannung und repräsentativ für die Biopartikel-Konzentration abzuleiten,

**dadurch gekennzeichnet, dass**

das magnetische Messsystem konfiguriert ist, die tatsächliche Amplitude des angelegten konstanten Gleichstrom-Magnetfelds so zu wählen, dass sie sich in unmittelbarer Umgebung (218, 210) einer Optimum-Amplitude des permanenten Gleichstrom-Magnetfelds befindet, das in einem Satz von zwei Optimum-Werten H1 und H2 des permanenten Gleichstrom-Magnetfelds umfasst ist, wobei jeder Optimum-Wert H1, H2 den absoluten Wert einer

ersten Größe $H \cdot \dfrac{\partial V_{MR}}{\partial H}$ lokal maximiert, wobei H die Amplitude des angelegten Magnetfelds ist und $\dfrac{\partial V_{MR}}{\partial H}$ die Ableitung der Differenzspannung in Bezug auf das Magnetfeld an dem angelegten Feld H ist.

2. Magnetisches Messsystem nach Anspruch 1, wobei der Durchmesser der Umgebung (218, 210) um die Optimum-Amplitude H1 und H2 des permanenten Gleichstrom-Magnetfelds kleiner ist als ein erster vorbestimmter Grenzwert, so dass ein erstes Verhältnis des absoluten Werts für die erste Größe $H \cdot \dfrac{\partial V_{MR}}{\partial H}$ bei der tatsächlichen Amplitude des angelegten konstanten Gleichstrom-Magnetfelds gegenüber dem absoluten Maximum-Wert der Größe

$H \cdot \dfrac{\partial V_{MR}}{\partial H}$ an der entsprechenden Optimum-Amplitude H1 oder H2 kleiner als minus 3 dB ist.

3. Messsystem nach einem der Ansprüche 1 bis 2, wobei die lokale Optimum-Amplitude H2 des permanenten Gleichstrom-Magnetfelds, das der tatsächlichen Amplitude des angelegten konstanten Gleichstrom-Magnetfelds am nächsten ist, unter den zwei Optimum-Amplituden H1, H2 des Satzes als die lokale Optimum-Amplitude gewählt wird, die eine zweite Größe $H \cdot \dfrac{\partial V_{MR}}{\partial H} / BN(H)$ maximiert, die festgelegt ist als die erste Größe $H \cdot \dfrac{\partial V_{MR}}{\partial H}$ zu dem Rauschniveau nach Barkhausen BN(H) an dem angelegten Feld, wobei das Rauschen nach Barkhausen proportional zu $\dfrac{dM}{\partial H}(H))$ ist, worin M die Magnetisierung des magnetischen Sensors ist.

4. Magnetisches Messsystem nach einem der Ansprüche 1 bis 3, wobei der Durchmesser der Umgebung um die Optimum-Amplitude H1 oder H2 des permanenten Gleichstrom-Magnetfelds kleiner ist als ein zweiter vorbestimmter Grenzwert, so dass ein zweites Verhältnis des absoluten Werts einer zweiten Größe $H \cdot \dfrac{\partial V_{MR}}{\partial H} / BN(H)$ an der tatsächlichen Amplitude des angelegten konstanten Gleichstrom-Magnetfelds zu dem absoluten Maximum-Wert der zweiten Größe $H \cdot \frac{\partial V_{MR}}{\partial H} / BN(H)$ an der entsprechenden Optimum-Amplitude H1 oder H2 kleiner ist als minus 3 dB, wobei die zweite Größe $H \cdot \frac{\partial V_{MR}}{\partial H} / BN(H)$ festgelegt ist als das Verhältnis der ersten Größe $H \cdot \frac{\partial V_{MR}}{\partial H}$ zu dem Rauschniveau nach Barkhausen BN(H) an dem angelegten Feld, wobei das Rauschen nach Barkhausen proportional zu $\frac{dM}{\partial H}(H))$ ist, worin M die Magnetisierung des magnetischen Sensors ist.

5. Magnetisches Messsystem nach einem der Ansprüche 1 bis 4, umfassend ferner eine magnetische AC-Wechselfeldquelle (314; 316) zum Anlegen eines tatsächlichen magnetischen AC-Wechselfelds an der Magnetspur (24) bei einer vorbestimmten Amplitude und einer vorbestimmten Frequenz, wobei das angelegte tatsächliche magnetische AC-Wechselfeld kollinear zu dem tatsächlichen konstanten Gleichstrom-Magnetfeld ist, das über das Magnetelement (18) angelegt wird.

6. Magnetisches Messsystem nach Anspruch 5, wobei die AC-Wechselfeldquelle (314; 316) entweder eine externe Magnetfeldquelle ist, einschließend mindestens eine Spule (318) und eine Spulen-Stromquelle (317) zur Versorgung einer Wechselstromquelle, oder eine elektrische Wechselstrom-Vorspannungsstromquelle (334), die konfiguriert ist, um einen AC-Wechsel-Vorspannungsstrom in die Magnetspur (24) einzuspeisen, wobei die Magnetspur (24) dementsprechend durch Selbstinduktion das angelegte AC-Wechselfeld erzeugt.

7. Magnetisches Messsystem nach einem der Ansprüche 1 bis 6, wobei das Magnetelement (18) ein Satz ist, der mindestens eine Magnetspule oder einen Permanentmagneten umfasst.

8. Magnetisches Messsystem nach einem der Ansprüche 1 bis 7, wobei die Magnetspur (24) ausgewählt wird in der Gruppe bestehend aus einer Kreuzspur, einer Ringspur und einer Mehrfach-Ringspur.

9. Magnetisches Messsystem nach einem der Ansprüche 1 bis 8, wobei die Magnetspur (24) eine Ringspur ist und die Zahl der Ringe zwischen 1 und 17 beträgt.

10. Magnetisches Messsystem nach einem der Ansprüche 1 bis 9, wobei die Magnetspur (24) in einem Aufbau hergestellt ist, der ausgewählt ist in der Gruppe bestehend aus einem dreilagigen Aufbau, einschließend einen ferromagnetischen Film, ein Metall und einen antiferromagnetischen Film, speziell Ta/NiFe/Cu/IrMn/Ta, einen zweilagigen Aufbau, speziell Ta/NiFe/IrMn/Ta, und ein Spin-Ventil, speziell Ta/NiFe/Cu/NiFe/IrMn/Ta.

11. Magnetisches Messsystem nach einem der Ansprüche 1 bis 10, wobei die Vorrichtung zur Signalverarbeitung (20; 320) einen eigenständigen Prozessor (48) umfasst, wenn kein magnetisches Wechselfeld an die Magnetspur (24) angelegt ist, und einen Prozessor (48) und eine Verriegelungseinheit (326) umfasst, wenn ein magnetisches Wechselfeld an die Magnetspur (24) angelegt ist; und die Vorrichtung zur Signalverarbeitung (320) konfiguriert ist, um aus der Differenzspannung $V_{MR}$ die erste oder zweite Harmonische der Differenzspannung zu extrahieren, wenn ein AC-Wechselfeld an die Magnetspur (24) angelegt wird; und die Vorrichtung zur Signalverarbeitung (320) konfiguriert ist, um die mindestens eine physikalische Größe basierend auf der ersten oder zweiten Harmonischen zu bestimmen, wenn die elektrische Vorspannungsstromquelle (14) einen AC-Wechsel-Vorspannungsstrom in die Magnetspur (24) einspeist oder wenn ein externes magnetisches Wechselfeld kollinear mit dem externen konstanten Gleichstrom-Magnetfeld angelegt wird.

12. Magnetisches Messverfahren zum Messen niedriger Konzentrationen spezifischer Biopartikel, die in einer gegebenen Lösung enthalten sind, und/oder zum quantitativen Bestimmen der Wechselwirkungen spezifischer Biopartikel, die in einer gegebenen Lösung enthalten sind, speziell in Lösungen mit Konzentrationen im Bereich von Nanomol bis Pikomol, wobei das magnetische Messverfahren die Schritte umfasst:

- Bereitstellen (404) eines magnetischen Messsystems (2), das zum Messen einer Konzentration von Biopartikeln durch einen empfindlichen Nachweis von Magnetpartikelmarkierungen konfiguriert ist, wobei das magnetische Messsystem (2) umfasst:
- ein Aptamer basierten magnetisch-hybriden AMR/PHR-Sensor (12), einschließend:

• eine vorbereitete aktive Oberfläche (22), auf der mindestens ein magnetisches Partikel (28) durch eine Aptamer-Struktur vom Sandwich-Typ (27), die ein eingefangenes unitäres Biopartikel einschließt, dicht gebunden ist oder dazu bereit ist, wobei die aktive Oberfläche eine Magnetspur (24) mit einer geschlossenen Schleifenform einschließt,
• einen ersten Stromanschluss (30) und einen zweiten Anschluss (32), die ein Paar zueinander gegenüberstehender Stromanschlüsse (30, 32) bilden und mit der Magnetspur (24) mit geschlossener Schleifenform in Kontakt stehen,
• einen ersten Spannungsanschluss (34) und einen zweiten Spannungsanschluss (36), die ein Paar zueinander gegenüberstehender Spannungsanschlüsse bilden und mit der Magnetspur (24) mit geschlossener Schleifenform in Kontakt stehen,

- Injizieren (406) eines Vorspannungsstroms in die Magnetspur (24) unter Verwendung der elektrischen Quelle (14), die eine elektrische Vorspannungs-Stromquelle (14) ist, die zwischen dem ersten und dem zweiten Stromanschluss (30, 32) und verbunden ist, und
- Anlegen (408) eines tatsächlichen konstanten Gleichstrom-Magnetfelds an die Magnetspur (24) bei einer vorbestimmten Amplitude, indem ein Magnetelement (18) verwendet wird;
- Messen (410) einer Differenzspannungs-$V_{MR}$ zwischen dem Paar von Spannungsanschlüssen (34, 36), indem eine Vorrichtung zur Spannungsmessung (16) verwendet wird, die zwischen dem ersten und dem zweiten Stromanschluss (30, 32) und verbunden ist,
- Bestimmen (412) mindestens einer physikalischen Größe, die auf der Differenzspannung basiert und repräsentativ ist für die Biopartikel-Konzentration, indem eine Vorrichtung zur Signalverarbeitung (20) verwendet wird:

wobei das magnetische Messverfahren **dadurch gekennzeichnet ist, dass** die tatsächliche Amplitude des angelegten konstanten Gleichstrom-Magnetfelds sich in unmittelbarer Umgebung einer Optimum-Amplitude des permanenten Gleichstrom-Magnetfelds befindet, die in einem Satz von zwei optimalen Werten H1, H2 des permanenten Gleichstrom-Magnetfelds enthalten ist, wobei jeder optimale Wert H1, H2 lokal den absoluten Wert einer ersten

Größe $H \cdot \dfrac{\partial V_{MR}}{\partial H}$ , worin H die Amplitude des angelegten Magnetfelds ist und $\dfrac{\partial V_{MR}}{\partial H}$ die Ableitung der Differenzspannung in Bezug auf das Magnetfeld an dem angelegten Feld H ist.

13. Magnetisches Messverfahren nach Anspruch 12, umfassend einen Schritt (414) des Aufbringens eines Tropfens einer magnetischen Lösung, die mit Streptavidin beschichtete magnetische Nanopartikel enthält, auf die vorbereitete aktive Oberfläche, ausgeführt
entweder vor dem Schritt des Bereitstellens des magnetischen Messsystems, wenn die Konzentration der Biopartikel gemessen wird, oder
nach dem Start der Schritte (406, 408) des Injizierens eines Vorspannungsstroms in die Magnetspur (24) und Anlegen eines Gleichspannungs-Magnetfelds auf die Magnetspur und nach dem Start des Schritts (410) des Messens einer Differenzspannung $V_{MR}$, wenn die Wechselwirkungen der spezifischen Bio-Moleküle quantitativ bestimmt werden.

14. Magnetisches Messverfahren nach einem der Ansprüche 12 bis 13, wobei die lokale Optimum-Amplitude H2 des permanenten Gleichstrom-Magnetfelds, die sich der tatsächlichen Amplitude des angelegten Gleichstrom-Magnetfelds am nächsten befindet, unter den zwei Optimum-Amplituden H1, H2 des Satzes als die lokale Optimum-Amplitude ausgewählt wird, die eine zweite Größe $H \cdot \dfrac{\partial V_{MR}}{\partial H} / BN(H)$ maximiert, die festgelegt ist als die erste Größe $H \cdot \dfrac{\partial V_{MR}}{\partial H}$ zu dem Rauschniveau nach Barkhausen BN(H) an dem angelegten Feld, wobei das Rauschen nach Barkhausen proportional zu $\dfrac{dM}{\partial H}(H))$ ist, worin M die Magnetisierung des magnetischen Sensors ist.

**15.** Magnetisches Messverfahren nach einem der Ansprüche 12 bis 14, umfassend den Schritt (416) zum Vorbereiten der aktiven Oberfläche (22) durch

- Modifizieren der aktiven Oberfläche mit Thiol-markiertem primären Aptamer
- Aufbringen eines Tropfens einer Lösung spezifischer Biopartikel in einer zu bestimmenden Konzentration auf die primär immobilisierte Oberfläche, wobei das Volumen des Tropfens a priori bekannt ist, sodann
- Verwenden von Biotin-markierten sekundären Aptameren, um an die Biopartikel zu binden.

**16.** Magnetisches Messverfahren nach einem der Ansprüche 12 bis 15 zum quantitativen Bestimmen der Wechselwirkungen von spezifischen Biopartikeln, die in einer gegebenen Lösung enthalten sind, umfassend einen zusätzlichen Signalverarbeitungsschritt (418), einschließend die Schritte:

- Bestimmen (420) der Kinetik des Bindens der magnetischen Partikel an die spezifischen Biopartikel, die auf der aktiven Oberfläche eingefangen und immobilisiert sind, aus der zeitlichen Entwicklung des gemessenen Magnetsignals, sodann
- Bestimmen (422) einer Sättigungszeit aus der Bindungskinetik der magnetischen Partikel;
- aus der Sättigungszeit und einer Modellfunktion, Berechnen eines mittleren Abstands, der die Biopartikel in ihrer Lösung trennt, und quantitatives Bestimmen (424) der Wechselwirkungen von spezifischen Biopartikeln, die sich in eine Coulomb-Wechselwirkung langer Reichweite und eine Van-der-Wals-Wechselwirkung kurzer Reichweite unterteilt.

## Revendications

**1.** Système de mesure magnétique permettant de mesurer une concentration de bio-particules spécifiques par le biais d'une détection sensible de marqueurs de particules magnétiques comprenant :

- un capteur magnéto-résistif/magnéto-résistif Hall plan anisotrope hybride magnétique à base d'aptamère (AMR/PHR) (12) présentant :

  • une surface active (22) sur laquelle au moins une particule magnétique (28) est prête à être ou est étroitement liée à travers une structure aptamère de type sandwich (27) qui inclut une bio-particule unitaire capturée, la surface active incluant une piste magnétique (24) avec une forme de boucle fermée,
  • une première borne de courant (30) et une seconde borne (32), formant une paire de bornes de courant (30, 32) faisant face l'une à l'autre et en contact avec la piste magnétique à boucle fermée (24),
  • une première borne de tension (34) et une seconde borne de tension (36) formant une paire de bornes de tension faisant face l'une à l'autre et en contact avec la piste magnétique à boucle fermée (24),

- une source de courant de polarisation électrique (14) configurée pour injecter un courant de polarisation dans la piste magnétique (24), la source électrique (14) étant raccordée entre la première et la seconde borne de courant (30, 32), et
- un dispositif de mesure de tension (16) raccordé entre la première et la seconde borne de tension (34, 36) mesurant une tension différentielle $V_{MR}$ entre la paire de bornes de tension (34, 36),
- un élément magnétique (18) permettant d'appliquer un champ magnétique constant DC effectif sur la piste magnétique à une amplitude prédéterminée ;
- un dispositif de traitement de signal (20 ; 320) configuré pour déduire au moins une quantité physique sur la base de la tension différentielle et représentative de la concentration de bio-particules **caractérisé en ce que** le système de mesure magnétique est configuré pour choisir l'amplitude effective du champ magnétique constant DC appliqué afin qu'elle soit située à proximité (218, 210) d'une amplitude optimale du champ magnétique DC permanent compris dans un ensemble de deux valeurs optimales H1, H2 du champ magnétique constant DC, chaque valeur optimale H1, H2 optimisant localement la valeur absolue d'une première quantité

$$H \cdot \frac{\partial V_{MR}}{\partial H}$$

, où H est l'amplitude du champ magnétique appliqué et $\dfrac{\partial V_{MR}}{\partial H}$ est la dérivée de la tension différentielle relativement au champ magnétique au niveau du champ appliqué H.

2. Système de mesure magnétique selon la revendication 1, dans lequel :
le diamètre de la proximité (218, 210) entourant l'amplitude optimale H1 et H2, du champ magnétique DC permanent est inférieur à un premier seuil prédéterminé de sorte qu'un premier rapport entre la valeur absolue de la première quantité $H \cdot \dfrac{\partial V_{MR}}{\partial H}$ à l'amplitude effective du champ magnétique constant DC appliqué et la valeur absolue maximale de la quantité $H \cdot \dfrac{\partial V_{MR}}{\partial H}$ à l'amplitude optimale correspondante H1 ou H2 est inférieur à moins 3 dB.

3. Système de mesure selon l'une quelconque des revendications 1 à 2, dans lequel l'amplitude optimale locale H2 du champ magnétique DC permanent le plus proche de l'amplitude effective du champ magnétique constant DC appliqué est choisie parmi les deux amplitudes optimales H1, H2 de l'ensemble comme l'amplitude optimale locale qui maximise une seconde quantité $H \cdot \dfrac{\partial V_{MR}}{\partial H} / BN(H)$ définie comme le rapport entre la première quantité $H \cdot \dfrac{\partial V_{MR}}{\partial H}$ et le niveau de bruit Barkhausen BN(H) au champ appliqué, le bruit Barkhausen étant proportionnel à $\dfrac{dM}{\partial H}(H))$, où M est la magnétisation du capteur magnétique.

4. Système de mesure selon l'une quelconque des revendications 1 à 3, dans lequel :
le diamètre de la proximité entourant l'amplitude optimale H1 ou H2 du champ magnétique DC permanent est inférieur à un second seuil prédéterminé de sorte qu'un second rapport entre la valeur absolue d'une seconde quantité $H \cdot \dfrac{\partial V_{MR}}{\partial H} / BN(H)$ à l'amplitude effective du champ magnétique constant DC appliqué et la valeur absolue maximale de la seconde quantité $H \cdot \dfrac{\partial V_{MR}}{\partial H} / BN(H)$ à l'amplitude optimale correspondante H1 ou H2 est inférieur à moins 3 dB, la seconde quantité $H \cdot \dfrac{\partial V_{MR}}{\partial H} / BN(H)$ étant définie comme le rapport entre la première quantité $H \cdot \dfrac{\partial V_{MR}}{\partial H}$ et le niveau de bruit Barkhausen BN(H) au champ appliqué, le bruit Barkhausen étant proportionnel à $\dfrac{dM}{\partial H}(H))$, où M est la magnétisation du capteur magnétique.

5. Système de mesure magnétique selon l'une quelconque des revendications 1 à 4,
comprenant en outre une source magnétique de champ AC (314 ; 316) permettant d'appliquer un champ magnétique alternatif AC effectif sur la piste magnétique (24) à une amplitude prédéterminée et à une fréquence prédéterminée, le champ magnétique alternatif AC effectif appliqué étant colinéaire avec le champ magnétique DC constant effectif appliqué par l'élément magnétique (18).

**6.** Système de mesure magnétique selon la revendication 5, dans lequel la source magnétique de champ AC (314, 316) est

soit une source de champ magnétique externe incluant au moins une bobine (318) et une source de courant de bobine (317) permettant d'alimenter une source de courant AC,

soit une source de courant de polarisation AC électrique (334), configurée pour injecter un courant de polarisation AC alternatif dans la piste magnétique (24), la piste magnétique (24) générant en conséquence par auto-induction le champ AC appliqué.

**7.** Système de mesure magnétique selon l'une quelconque des revendications 1 à 6, dans lequel l'élément magnétique (18) est un ensemble comprenant au moins une bobine magnétique ou un aimant permanent.

**8.** Système de mesure magnétique selon l'une quelconque des revendications 1 à 7, dans lequel la piste magnétique (24) est choisie dans le groupe constitué d'une piste croisée, d'une piste annulaire et d'une piste multi-anneaux.

**9.** Système de mesure magnétique selon l'une quelconque des revendications 1 à 8, dans lequel la piste magnétique (24) est une piste annulaire, le nombre d'anneaux étant compris entre 1 et 17.

**10.** Système de mesure magnétique selon la revendication 1 à 9, dans lequel la piste magnétique (24) est réalisée dans une structure choisie dans le groupe constitué d'une structure à trois couches incluant un film ferromagnétique, un film métallique et antiferromagnétique, en particulier Ta/NiFe/Cu/IrMn/Ta, une structure bicouches, en particulier Ta/NiFe/IrMn/Ta, et une vanne de spin, en particulier Ta/NiFe/Cu/NiFe/IrMn/Ta.

**11.** Système de mesure magnétique selon l'une quelconque des revendications 1 à 10, dans lequel :

le dispositif de traitement de signal (20 ; 320) comprend un processeur autonome (48) quand aucun champ magnétique AC n'est appliqué sur la piste magnétique (24), et comprend un processeur (48) et une unité d'enclenchement (326) quand un champ magnétique AC est appliqué sur la piste magnétique (24) ; et

le dispositif de traitement de signal (320) est configuré pour extraire de la tension différentielle $V_{MR}$ la première ou seconde harmonique de la tension différentielle quand un champ magnétique AC est appliqué sur la piste magnétique (24) ; et

le dispositif de traitement de signal (320) est configuré pour déterminer ladite au moins une quantité physique sur la base de ladite première ou seconde harmonique, quand la source de courant de polarisation électrique (14) injecte un courant de polarisation AC alternatif dans la piste magnétique (24) ou quand un champ magnétique AC extérieur est appliqué de manière colinéaire avec le champ magnétique DC constant extérieur.

**12.** Procédé de mesure magnétique permettant de mesurer de faibles concentrations de bio-particules spécifiques contenues dans une solution donnée et/ou de quantifier les interactions de bio-particules spécifiques contenues dans une solution donnée, en particulier dans des solutions présentant des concentrations comprises entre des nanomoles et des picomoles,

le procédé de mesure magnétique comprenant les étapes consistant à :

- fournir (404) un système de mesure magnétique (2) configuré pour mesurer une concentration de bio-particules à travers une détection sensible de marqueurs de particules magnétiques, le système de mesure magnétique (2) comprenant :

- un capteur AMR/PHR hybride magnétique à base d'aptamère (12) incluant :

• une surface préparée (22) sur laquelle au moins une particule magnétique (28) est prête à être ou est étroitement liée à travers une structure aptamère de type sandwich qui inclut une bio-particule unitaire capturée, la surface active incluant une piste magnétique (24) avec une forme en boucle fermée,
• une première borne de courant (30) et une seconde borne (32), formant une paire de bornes de courant (30, 32) en face l'une de l'autre et en contact avec la piste magnétique à boucle fermée (24),
• une première borne de tension (34) et une seconde borne de tension (36) formant une paire de bornes de tension l'une face à l'autre et en contact avec la piste magnétique à boucle fermée (24),

- injecter (406) un courant de polarisation dans la piste magnétique (24) en utilisant la source électrique (14) qui est une source de courant de polarisation électrique (14) connectée entre la première et la seconde borne de courant (30, 32) ; et
- appliquer (408) un champ magnétique constant DC effectif sur la piste magnétique (24) à une amplitude prédéterminée en utilisant un élément magnétique (18) ;
- mesurer (410) une tension différentielle $V_{MR}$ entre la paire de bornes de tension (34, 36) en utilisant un dispositif de mesure de tension (16) raccordé entre la première et la seconde borne de tension (34, 36) ;
- déterminer (412) au moins une quantité physique basée sur la tension différentielle et représentative de la concentration de bio-particules en utilisant un dispositif de traitement de signal (20) ;

ledit procédé de mesure magnétique étant **caractérisé en ce que** :
l'amplitude effective du champ magnétique constant DC appliqué est étroitement située à proximité d'une amplitude optimale du champ magnétique DC permanent compris dans un ensemble de deux valeurs optimales H1, H2 du champ magnétique DC permanent, chaque valeur optimale H1, H2 maximisant localement la valeur absolue d'une

première quantité $H \cdot \dfrac{\partial V_{MR}}{\partial H}$, où H est l'amplitude du champ magnétique appliqué et $\dfrac{\partial V_{MR}}{\partial H}$ est la dérivée de la tension différentielle relativement au champ magnétique au champ appliqué H.

**13.** Procédé de mesure magnétique selon la revendication 12, comprenant une étape (414) consistant à appliquer sur la surface active préparée une goutte d'une solution magnétique contenant des nanoparticules magnétiques revêtues de streptavidine qui est exécutée :

soit avant l'étape consistant à fournir le système de mesure magnétique lors de la mesure de la concentration des bio-particules,
soit après le début des étapes (406, 408) consistant à injecter un courant de polarisation dans la piste magnétique (24) et à appliquer un champ magnétique constant DC sur la piste magnétique et après le démarrage de l'étape (410) de mesure d'une tension différentielle $V_{MR}$ lors de la quantification des interactions des biomolécules spécifiques.

**14.** Procédé de mesure magnétique selon l'une quelconque des revendications 12 à 13, dans lequel l'amplitude optimale locale H2 du champ magnétique DC permanent le plus proche de l'amplitude effective du champ magnétique constant DC appliqué est sélectionnée parmi les deux amplitudes optimales H1, H2 de l'ensemble comme l'amplitude optimale locale qui maximise une seconde quantité $H \cdot \dfrac{\partial V_{MR}}{\partial H} / BN(H)$ définie comme le rapport entre la

première quantité $H \cdot \dfrac{\partial V_{MR}}{\partial H}$ et le niveau de bruit Barkhausen BN(H) au champ appliqué, le bruit Barkhausen

étant proportionnel à $\dfrac{dM}{\partial H}(H))$, où M est la magnétisation du capteur magnétique.

**15.** Procédé de mesure magnétique selon l'une quelconque des revendications 12 à 14, comprenant une étape (416) consistant à préparer la surface active (22) :

- en modifiant la surface active avec un aptamère primaire marqué au thiol, puis
- en appliquant une goutte d'une solution de bio-particules spécifiques à une concentration à déterminer à la surface immobilisée primaire, le volume de la goutte étant a priori connu, puis
- en utilisant des aptamères secondaires marqués à la biotine pour se lier avec les bio-particules.

**16.** Procédé de mesure magnétique selon l'une quelconque des revendications 12 à 15, permettant de quantifier les interactions de bio-particules spécifiques contenues dans une solution donnée comprenant une étape de traitement de signal supplémentaire (418) incluant les étapes consistant à :

- déterminer (420) la cinétique de la liaison des particules magnétiques aux bio-particules spécifiques capturées et immobilisées sur la surface active à partir de l'évolution temporelle du signal magnétique mesuré ; puis

- déterminer (422) un temps de saturation à partir de la cinétique de liaison des particules magnétiques ;
- à partir du temps de saturation et d'une fonction de modèle, calculer une distance moyenne séparant les bio-particules dans leur solution et quantifier (424) les interactions de bio-particules spécifiques qui se répartissent dans une interaction de Coulomb à long terme et une interaction de Van de Waals à court terme.

FIG.1

| | |
|---|---|
| ● | Ion oxide |
| ✕ | Streptadivin |
| ⌇▪ | Biotinated aptamer |
| ◆ | Thrombin |
| ⌇ | Thiolated aptamer |

FIG.2

FIG.3

FIG.4

Ratio of $BN_1:BN_2 = 18$

=> Increase signal to noise ratio 18 times if operate at $H_2$

FIG.5

FIG.6

FIG.7

402

FIG.8

402

416

404

406

408

410

414

412

418

420

422

424

FIG.9

FIG.10

FIG.11

FIG.12

FIG.13

FIG.14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2015168507 A1

### Non-patent literature cited in the description

- **BIN DENG et al.** Aptamer binding assays for proteins: the thrombin example - A review. *Analytica Chimica Acta,* 2014, vol. 837, 1-15
- Hybrid AMR/PHR ring sensor. **SUNJONG O et al.** Solid State Communications. Pergamon, GB, 29 May 2011, vol. 151, 1248-151
- Analytes kinetics in lateral flow membrane analyzed by cTNI monitoring using magnetic method. **SUNJONG OH et al.** Sensors ad Actuators B: Chemical International : International journal devoted to research and development of physical and chemical transducers. Elsevier BV, 19 August 2011, vol. 160, 747-752
- **P. SINHA et al.** Planar Hall magnetoresistive aptasensor for thrombin detection. *Biosensors and Bioelectronics,* 2014, vol. 59, 140-144